(19) Europäisches Patentamt
European Patent Office
Office européen des brevets

(11) Veröffentlichungsnummer: **0 123 297 B1**

(12) # EUROPÄISCHE PATENTSCHRIFT

(45) Veröffentlichungstag der Patentschrift: **03.08.88**

(51) Int. Cl.⁴: **C 08 F 230/02**, C 08 F 246/00, C 04 B 24/26, C 09 D 3/74, A 61 L 15/00, D 06 P 1/52

(21) Anmeldenummer: **84104477.9**

(22) Anmeldetag: **19.04.84**

(54) **Vernetzte hydrophile Copolymerisate, ihre Herstellung und Verwendung.**

(30) Priorität: **22.04.83 DE 3314569**

(43) Veröffentlichungstag der Anmeldung: **31.10.84 Patentblatt 84/44**

(45) Bekanntmachung des Hinweises auf die Patenterteilung: **03.08.88 Patentblatt 88/31**

(84) Benannte Vertragsstaaten: **AT BE CH DE FR GB IT LI NL SE**

(56) Entgegenhaltungen: **EP - A - 0 032 663**

(73) Patentinhaber: **CASSELLA Aktiengesellschaft, Hanauer Landstrasse 526, D-6000 Frankfurt am Main 61 (DE)**

(72) Erfinder: **Engelhardt, Friedrich, Dr., Hünfelderstrasse 20, D-6000 Frankfurt/Main 61 (DE)**
Erfinder: **Kühlein, Klaus, Dr., Fasanenstrasse 41, D-6233 Kelkheim (DE)**
Erfinder: **Balzer, Juliane, Im Trutz 51, D-6000 Frankfurt/Main 61 (DE)**
Erfinder: **Dürsch, Walter, Dr., In der Braubach 4, D-6240 Königstein 4 (DE)**
Erfinder: **Kleiner, Hans-Jerg, Dr., Altkönigstrasse 11a, D-6242 Kronberg 2 (DE)**

(74) Vertreter: **Urbach, Hans-Georg, Dr., Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Es ist bekannt (US-P 4 085 167, DE-AS 1 135 173), durch Copolymerisation wasserlöslicher Monomeren mit mehrfach olefinisch ungesättigten Verbindungen Copolymere herzustellen, welche in Abhängigkeit von der Monomerenzusammensetzung imstande sind, unterschiedliche Mengen Wasser unter Aufquellen zu absorbieren, wobei in der Regel das Quellvermögen mit abnehmender Vernetzermenge (polyolefinische Komponente) ansteigt. Derartig vernetzte hydrophile Copolymere, insbesondere auf Basis Acrylsäure-Methacrylsäure werden z.B. als Verdickungsmittel vielfältig eingesetzt (Druckpasten, Kosmetik). Ein weiteres Einsatzgebiet für derartige hochquellfähige Polymere ist der Hygienesektor, wobei derartige Polymerisate infolge ihrer hohen Saugfähigkeit angewendet werden.

Weiterhin finden vernetzte hydrophile Polymere zur Verfestigung und Wasserretention in Böden Verwendung.

Es wurde nun gefunden, dass vernetzte hydrophile Copolymerisate, die 0,01 bis 30 Gew.% Brückenglieder der Formel I

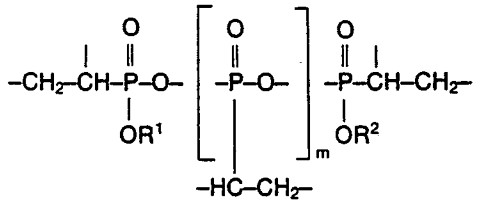

(I)

worin $R^1$ und $R^2$ unabhängig voneinander Wasserstoff oder Alkyl mit 1 bis 4 C-Atomen bedeuten und m Werte von 0 bis 6 besitzt, und 99,99 bis 70 Gew.% Grundkettenbausteine der Formel III

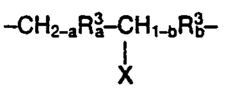

(III)

worin

$R^3$ Wasserstoff oder Methyl ist, a und b jeweils die Werte 0 oder 1 haben und die Summe a+b 0 oder 1 ist und X die Karbonamidgruppe $-CONH_2$; eine Gruppe der Formel IV

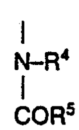

(IV)

worin $R^4$ und $R^5$ unabhängig voneinander für Wasserstoff, Methyl oder Ethyl oder gemeinsam für Trimethylen oder Pentamethylen stehen; Carboxyl oder dessen Salz mit einem Kation $M^{\oplus}$; Alkoxycarbonyl mit 1 bis 6 Kohlenstoffatomen im Alkylteil; Hydroxyalkoxycarbonyl mit 1 bis 3 Kohlenstoffatomen im Hydroxyalkylteil; N-Methylolcarbonamid der Formel $HOCH_2NH-CO-$, dessen Methylolgruppe gegebenenfalls mit Alkanolen mit 1 bis 4 Kohlenstoffatomen verethert sein kann; Alkanoylamino mit 1 bis 4 Kohlenstoffatomen, das gegebenenfalls durch Methylol oder Alkyl mit 1 bis 4 Kohlenstoffatomen N-substituiert sein kann; Cyan; gegebenenfalls durch Alkyl mit 1 bis 4 Kohlenstoffatomen, Alkoxy mit 1 oder 2 Kohlenstoffatomen, Halogen, Trifluormethyl oder Nitro substituiertes Phenyl oder Benzyl; Imidazolyl-(1); die Sulfonsäuregruppe; Sulfoalkylamidocarbonyl mit 1 bis 4 Kohlenstoffatomen im Alkylrest; die Phosphonsäuregruppe, wobei die Sulfonsäure- und Phosphonsäuregruppen auch in Form ihrer Salze mit einem Kation $M^{\oplus}$ vorliegen können; die Phosphonsäureestergruppe der Formel V

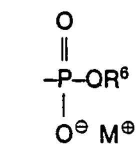

(V)

worin $R^6$ Alkyl mit 1 bis 4 C-Atomen ist; einen Rest der Formel VI

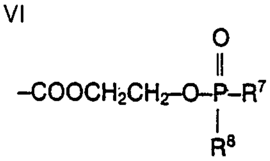

(VI)

worin $R^7$ und $R^8$ gleich oder verschieden sind und für Alkyl mit 1 bis 7 Kohlenstoffatomen stehen; einen Rest der Formel VII

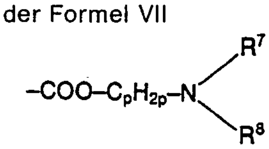

(VII)

worin $R^7$ und $R^8$ die oben angegebenen Bedeutungen haben und p für eine Zahl von 1 bis 4 steht; oder einen Rest der Formel VIII

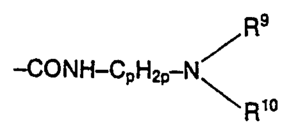

(VIII)

worin $R^9$ und $R^{10}$ gleich oder verschieden sind und für Alkyl mit 1 bis 4 Kohlenstoffatomen stehen und p für eine Zahl von 1 bis 4 steht; sowie die den Formeln VII und VIII entsprechenden quaternisierten Gruppen bedeuten, enthält, wobei sich das Kation $M^{\oplus}$ von einer wasserlöslichen Base ableitet, deren Stärke ausreicht, die genannten Sulfonsäure- bzw. Carboxylgruppen zu neutralisieren und die deren Hydrophilie nicht beeinträchtigt, spezielle, anwendungstechnisch sehr wertvolle Eigenschaften aufweisen.

Die erfindungsgemässen Copolymerisate besitzen im alkalischen, neutralen und schwach sauren Bereich ($pH \geqslant 2$) die Struktur eines Polymerennetzwerkes, welches bei pH-Werten $<2$, d.h. durch stärkere Säuren, aufgespalten wird. Sie sind daher in stärkeren wässrigen Säuren direkt löslich, in alkalischen bis schwach sauren wässrigen Lösungen bei pH-Werten über 2 jedoch nur quellbar; sie bieten somit die Möglichkeit eines exakt steuerbaren Abbaus im sauren pH-Bereich.

Die erfindungsgemässen neuen Polymeren eignen sich daher beispielsweise vorzüglich zur Herstellung von säurelöslichen Überzügen und Verkapselungsmaterialien.

Die Möglichkeit, durch Säureeinwirkung die

Struktur der erfindungsgemässen Copolymerisate gravierend zu verändern, dürfte darauf zurückzuführen sein, dass die vernetzenden Brückenglieder der Formel I im sauren pH-Bereich hydrolytisch spaltbar sind gemäss dem folgenden Reaktionsschema

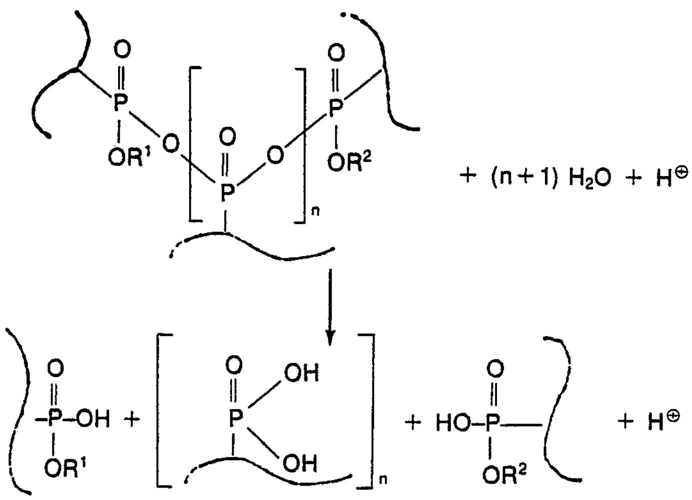

wobei das gequollene Polymere seine Gelstruktur verliert und in eine unvernetzte lösliche Polymerstruktur übergeht. (In obigem Schema symbolisiert das Zeichen ~ Grundketten des Polymerisats.)

Vorzugsweise bedeuten in den Brückengliedern der Formel I $R^1$ und $R^2$ unabhängig voneinander Wasserstoff oder Alkyl mit 1 oder 2 C-Atomen.

In den erfindungsgemässen vernetzten Copolymerisaten können die Brückenglieder der Formel I alle exakt die gleiche Struktur haben. Dies ist dann der Fall, wenn bei der Herstellung der Produkte eine einzige reine Vernetzersubstanz der Formel Ia

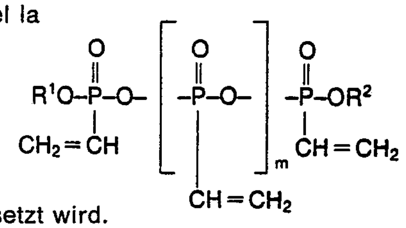

(Ia)

eingesetzt wird.

Erfindungsgemässe vernetzte Copolymerisate können jedoch auch mehrere Arten strukturell unterschiedlicher Brückenglieder enthalten. So können sich die Brückenglieder hinsichtlich der Bedeutung der Reste $R^1$ und $R^2$ und/oder des Baugruppenindexes m voneinander unterscheiden, insbesondere können die Baugruppenindices m in statistischer Verteilung Werte von 0 bis 6 annehmen.

Die Häufigkeit der Einzelwerte für m kann dabei jeweils zwischen 0 und 1 liegen, wobei als Häufigkeit der Anteil eines Wertes m bezogen auf alle vorhandenen Werte für m definiert ist. Haben beispielsweise in einem Produkt die Brückenglieder m-Werte zwischen 0 und 6, und weisen von je 100 Brückengliedern 35 einen m-Wert von 1, 25 einen m-Wert von 3, 20 einen m-Wert von 4 und 5 einen m-Wert von 6 auf, so ist die Häufigkeit für

$$m = 1 : \frac{35}{100} = 0{,}35 \text{ ; für } m = 3 : \frac{25}{100} = 0{,}25;$$

für $m = 4 : 0{,}2$ und für $m = 6 : 0{,}05$.

Die Aussage, dass in einem erfindungsgemässen Produkt die Häufigkeit eines speziellen m-Wertes 1 ist, bedeutet, dass dieses Produkt ausschliesslich Brückenglieder mit diesem speziellen m-Wert enthält; ist die Häufigkeit eines speziellen m-Wertes in einem erfindungsgemässen Copolymerisat 0, so heisst dies, dass in diesem Copolymerisat keine Brückenglieder mit diesem speziellen m-Wert vorkommen.

In der Praxis sind erfindungsgemässe Copolymerisate, die verschiedene vernetzende Brükkenglieder mit unterschiedlichen m-Werten enthalten, besonders vorteilhaft, weil sie bei guten anwendungstechnischen Eigenschaften wesentlich einfacher und damit kostengünstiger hergestellt werden können. Die für die Einführung der Brückenglieder in die Makromoleküle eingesetzten Vernetzersubstanzen der Formel Ia können nur auf relativ umständliche Weise als einheitliche Substanzen hergestellt werden. Einfach jedoch sind Gemische dieser Substanzen zu erhalten, z.B. nach einem in der EP-A-32 663 beschriebenen Verfahren. Solche Gemische sind in der angegebenen Europäischen Patentanmeldung durch die Formel

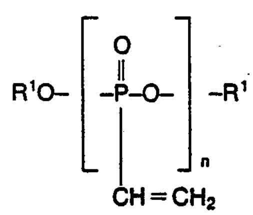

definiert worden, wobei $R^1$ Wasserstoff oder Alkyl mit 1–4 C-Atomen bedeutet und n in der Praxis Werte von 1 bis 8 annehmen kann.

Solche Gemische können tel quel ohne Nachteile zur Herstellung der erfindungsgemässen Produkte eingesetzt werden. Man erhält dann bevorzugte erfindungsgemässe vernetzte Copolymerisate, bei denen im Rahmen der statistischen Verteilung der Baugruppenindices m die Häufigkeit der einzelnen m-Werte mit der Häufigkeit der Werte für n in einem Rohprodukt der Herstellung von Verbindungen der Formel II

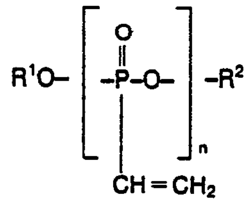 (II)

gemäss der EP-A-32 663, korreliert ist und zwar in der Weise, dass die Häufigkeit eines Wertes m gleich ist der Häufigkeit desjenigen Wertes n, der dem Zusammenhang $n = m + 2$ genügt.

Die einzelnen Arten der Brückenglieder sind dabei in den Makromolekülen der erfindungsgemässen Copolymerisate in der Regel statistisch verteilt.

Abweichungen von der rein statistischen Verteilung der Brückenglieder und auch der Monomeren in den Grundketten ändern erfahrungsgemäss nichts an der anwendungstechnischen Brauchbarkeit der erfindungsgemässen vernetzten Copolymerisate. Geringfügige Abweichungen von der rein statistischen Verteilung können bereits durch die unterschiedliche Reaktivität der Monomeren und Vernetzer zustande kommen.

Die erfindungsgemässen vernetzten Copolymerisate enthalten 0,01 bis 30 Gew.%, vorzugsweise 0,01 bis 6 Gew.%, insbesondere 0,05 bis 2 Gew.%, Brückenglieder der Formel I und 99,99 bis 70 Gew.%, vorzugsweise 99,99 bis 94 Gew.%, insbesondere 99,95 bis 98 Gew.%, Grundkettenbausteine. Hierbei sind in dem Gew.%-Anteil der Brükkenglieder auch die in den Vernetzern der Formel Ia vorhandenen Vinylgruppen einbezogen, die bei der Copolymerisation in die Grundketten eingebaut werden.

In den Grundkettenbausteinen der Formel III enthalten die für $R^4$ und/oder $R^5$ stehenden Alkoxycarbonylgruppen bevorzugt 1 bis 3 Kohlenstoffatome im Alkylteil.

Die für $R^6$, $R^7$ und/oder $R^8$ und $R^9$ und/oder $R^{10}$ stehenden Alkylreste enthalten bevorzugt 1 oder 2 Kohlenstoffatome.

Die tertiären Aminstrukturen in den Formeln VII und VIII können auch quaternisiert vorliegen. Zur Quaternisierung können beispielsweise Dimethylsulfat und Methylchlorid verwendet werden.

Ein für X stehender Phenylkern und der Phenylkern eines für X stehenden Benzylkerns sind entweder unsubstituiert, oder sie tragen 1 oder 2 Substituenten.

Bevorzugte Substituenten sind Chlor, Methyl und Ethyl. Einfachsubstitution kann in o-, m- oder p-Stellung zur Vinyl- oder Allylgruppe vorliegen. Zweifachsubstitution vorzugsweise in 2,4- oder 2,6-Stellung, aber auch in 2,5-, 3,5- oder 3,4-Stellung.

Bevorzugt sind, sofern X Phenyl oder Benzyl bedeutet, die im aromatischen Kern nur einfach substituierten und besonders bevorzugt die unsubstituierten Reste.

Um den gewünschten hydrophilen Charakter der erfindungsgemässen Copolymerisate zu erhalten, ist zu beachten, dass mindestens 70% der Grundkettenbausteine solche Reste X aufweisen, die hydrophilen Charakter besitzen und mindestens etwa 2%, vorzugsweise mindestens 7%, der Reste X saure Gruppen aufweisen bzw. deren Salze mit dem Kation $M^{\oplus}$.

Typische Gruppen, die hydrophilen Charakter haben, sind der Sulfonsäurerest oder Carboxyl sowie Gruppen X, die diese sauren Reste tragen, Carbonamid ($-CO-NH_2$) sowie dessen Methylolderivat, und die Gruppe der Formel IV.

Typische Gruppen ohne hydrophilen Charakter sind z.B. Cyan, Phenyl oder Benzyl.

Das Kation $M^+$ kann sich prinzipiell von jeder wasserlöslichen bekannten Base ableiten, deren Stärke ausreicht, die Sulfonsäuregruppen bzw. Carboxylgruppen der erfindungsgemässen vernetzten Copolymerisate zu neutralisieren und die deren Hydrophilie nicht beeinträchtigt. Die Auswahl kann somit in einfacher bekannter Weise erfolgen.

Zweckmässigerweise bedeutet jedoch $M^+$ ein Erdalkali- oder vorzugsweise ein Alkalikation, insbesondere ein Natrium- oder Kaliumkation, Ammonium oder ein von niederen aliphatischen Aminen abgeleitetes Kation. Niedere aliphatische Amine, von denen sich die Kationen $M^+$ ableiten können, sind primär, sekundär oder tertiär und weisen gegebenenfalls durch –OH-Gruppen substituierte Alkylgruppen mit 1 bis 4 C-Atomen auf. Bevorzugt sind solche, die mindestens einen β-Hydroxyethylrest enthalten, wie z.B. β-Aminoethanol, β-Dimethylamino-ethanol, Bis-(β-hydroxyethyl)-methylamin, Tris-(β-hydroxyethyl)-amin, Diethyl-β-hydroxyethylamin, Bis-(β-hydroxyethyl)-ethylamin.

Bevorzugt sind solche erfindungsgemässen vernetzten Copolymerisate, deren Grundketten aus Bausteinen der Formel IX

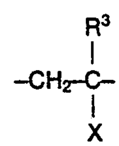 (IX)

aufgebaut sind, worin $R^3$ die oben angegebene Bedeutung hat und X die Karbonamidgruppe $-CONH_2$; eine Gruppe der Formel IV

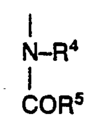 (IV)

worin $R^4$ und $R^5$ unabhängig voneinander für Wasserstoff, Methyl oder Ethyl oder gemeinsam für Trimethylen oder Pentamethylen stehen; Carboxyl oder dessen Alkali- oder Ammoniumsalze, Hydroxyalkoxycarbonyl mit 2 oder 3, vorzugsweise 2, C-Atomen im Hydroxyalkylteil; die Sulfonsäuregruppe; Sulfoalkylamidocarbonyl mit 1 bis 4 C-Atomen im Alkylrest, vorzugsweise eine Gruppe der Formel

$CO{-}NH{-}C(CH_3)_2{-}CH_2{-}SO_3^- \; M^+$

die Phosphonsäuregruppe, wobei die Sulfonsäure- und Phosphonsäuregruppen auch in Form ihrer Alkali- oder Ammoniumsalze vorliegen können; und die Phosphonsäureestergruppe der Formel V

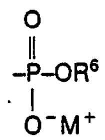 (V)

worin $R^6$ Alkyl mit 1 bis 4, vorzugsweise 1 oder 2, C-Atomen ist, bedeutet.

Weiterhin sind solche erfindungsgemässen vernetzten Copolymerisate bevorzugt, bei denen, bezogen auf die Gesamt-Gewichtsmenge der Grundketten, in 5 bis 70 Gew.%, insbesondere in 10 bis 65 Gew.%, der Grundkettenbausteine der Formel III X eine Sulfo- oder Sulfoalkylamidocarbonylgruppe mit 1 bis 4 C-Atomen im Alkylrest ist, in 0 bis 40 Gew.%, insbesondere in 0 bis 25 Gew.%, der Grundkettenbausteine der Formel III X eine Gruppe der Formel IV

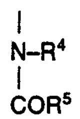 (IV)

ist und in 30 bis 95 Gew.%, insbesondere in 30 bis 80 Gew.% der Grundkettenbausteine der Formel III X eine der übrigen obengenannten Bedeutungen hat.

In der Regel haben in einem einzelnen Makromolekül die Reste X nicht mehr als 10, vorzugsweise nicht mehr als 6, verschiedene Bedeutungen.

Besonders bevorzugt sind solche erfindungsgemässen vernetzten Copolymerisate, in denen mehrere der obengenannten bevorzugten Merkmale vereinigt sind.

Die Herstellung wasserlöslicher Polymerisate, welche Sulfonsäuregruppen im Makromolekül eingebaut enthalten, ist bereits in zahlreichen Patenten sowie in der Fachliteratur ausführlich beschrieben worden. So ist z.B. die Synthese von Copolymeren der Vinylsulfonsäure mit Acrylamid und Vinylpyrrolidon in J. Polymer Sci., 38, 147 (1959) veröffentlicht worden.

Im DE-C-1 101 760 ist ein Verfahren zur Herstellung wasserlöslicher Copolymerisate aus Vinylsulfonsäure und Acrylnitril bzw. Methacrylnitril gegebenenfalls im Gemisch mit weiteren ethylenisch ungesättigten Verbindungen beschrieben worden. Copolymerisate aus Vinyl- bzw. Allylsulfonaten mit Acrylamid und Vinylamiden sind z.B. in der DE-B-2 444 108 beschrieben worden.

Wasserlösliche Copolymerisate, welche 2-Acrylamido-2-methyl-propansulfonsäure-(1), im folgenden mit AIBS abgekürzt, als Comonomeres enthalten, sind in den US-Patentschriften 3 953 342, 3 768 565, 3 907 927, 3 926 718, sowie in den DE-A-2 502 012 und 2 547 773 beschrieben.

Die erfindungsgemässen vernetzten Copolymerisate, soweit sie Reste der Formel IV enthalten, bei denen $R^4$ und $R^5$ gemeinsam Trimethylen oder Pentamethylen bedeuten, lassen sich in der aus dem Stand der Technik bekannten Weise, z.B. gemäss den Angaben der US-A-3 929 741 durch Umsetzung der Monomeren bei Temperaturen von etwa 10 bis 120 °C, vorzugsweise bei 40 bis 80 °C, in Gegenwart von geeigneten Polymerisationskatalysatoren herstellen.

Will man, um erfindungsgemässe vernetzte Copolymerisate herzustellen, die Gruppen der Formel IV enthalten, in denen $R^4$ und $R^5$ nicht gemeinsam für Tri- oder Pentamethylen stehen, unter analogen Bedingungen die Copolymerisation von AIBS, Styrol- oder Vinylsulfonsäure mit nicht ringgeschlossenen N-Vinylamiden der Formel IVa

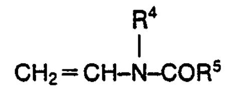 (IVa)

durchführen, so ist es erforderlich, die sauren Komponenten vor der Polymerisation durch Zusatz von Basen in die Salze mit dem Kation $M^+$ zu überführen. Die hierbei zweckmässigerweise eingesetzten Basen sind die Hydroxyde oder Salze der Kationen $M^+$ mit schwachen Säuren, wie z.B. Kohlensäure oder Phosphorsäure, oder im Falle von Aminenbasen $NH_3$ oder die freien Amine, die oben bereits im einzelnen genannt wurden.

Die Neutralisation der sauren Komponenten vor der Polymerisation ist aber auch bei der Copolymerisation von ringgeschlossenen Verbindungen IVa möglich und in der Regel sogar vorteilhaft.

Zweckmässigerweise werden somit zur Herstellung von je 100 Gewichtsteilen des Copolymerisats 0,01 bis 30 Gewichtsteile, vorzugsweise 0,01 bis 6, insbesondere 0,05 bis 2 Gewichtsteile eines Vernetzers der Formel Ia

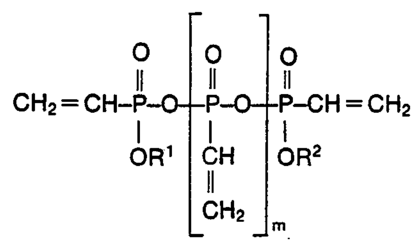 (Ia)

worin $R^1$, $R^2$ und m die oben angegebenen Bedeutungen haben, in reiner Form oder eine Mischung mehrerer Verbindungen dieser allgemeinen Formel mit 70 bis 99,99 Gewichtsteilen, vorzugsweise mit 94 bis 99,99, insbesondere 98 bis 99,95 Gewichtsteilen von Comonomeren der Formel IIIa

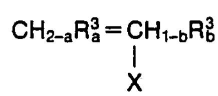 (IIIa)

und vorzugsweise von solchen der Formel IXa

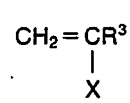 (IXa)

worin $R^3$, X, a und b die oben genannten Bedeutungen haben und zwischen a und b die oben

angegebene Beziehung besteht, copolymerisiert, wobei für den Fall, dass ein Comonomeres der Formel IIIa eingesetzt wird, in dem X eine Gruppe der Formel IV ist, worin $R^4$ und $R^5$ nicht gemeinsam eine Tri- oder Pentamethylengruppe bilden, obligatorisch, für den Fall, dass kein derartiges Comonomeres eingesetzt wird, gegebenenfalls durch Zusatz einer Base die sauren Gruppen neutralisiert werden und die Copolymerisation in an sich bekannter Weise eingeleitet und bei 10 bis 120 °C durchgeführt wird.

Besonders zweckmässig ist es, bei der Herstellung der erfindungsgemässen vernetzten Copolymerisate keine reine Verbindung der Formel Ia als Vernetzer einzusetzen, sondern ein Rohprodukt der Herstellung von Verbindungen der Formel II

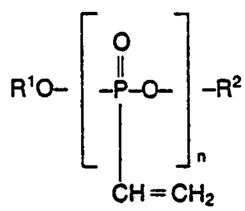

(II)

gemäss der EP-A-32 663.

In diesem Fall werden erfindungsgemässe vernetzte Copolymerisate erhalten, die mehrere verschiedene Brückenglieder der Formel I enthalten, wobei im Rahmen der statistischen Verteilung der Baugruppenindices m die Häufigkeit der einzelnen m-Werte mit der Häufigkeit der Werte für n in dem eingesetzten Rohprodukt in der Weise korreliert ist, dass die Häufigkeit eines Wertes m gleich ist der Häufigkeit desjenigen Wertes n, der dem Zusammenhang $n = m + 2$ genügt.

Bevorzugte erfindungsgemässe Copolymerisate werden auch erhalten, wenn in 5 bis 70 Gew.%, insbesondere in 10 bis 65 Gew.%, der Comonomeren der Formel IIIa X eine Sulfo- oder Sulfoalkylamidocarbonylgruppe mit 1 bis 4 C-Atomen im Alkylrest ist, in 0 bis 40 Gew.%, insbesondere in 0 bis 25 Gew.%, der Comonomeren der Formel IIIa X eine Gruppe der Formel IV ist und in 30 bis 95 Gew.%, insbesondere in 30 bis 80 Gew.% der Comonomeren der Formel IIIa X eine der übrigen oben genannten Bedeutungen hat.

Die Polymerisation kann als Gelpolymerisation, als Fällungspolymerisation oder in umgekehrter Emulsion ausgeführt werden.

Bei der Durchführung der Copolymerisation in einem mit Wasser mischbaren organischen Lösungsmittel arbeitet man unter den Bedingungen der Fällungspolymerisation. Hierbei fällt das Polymerisat direkt in fester Form an und kann durch Abdestillieren des Lösungsmittels oder Absaugen und Trocknen isoliert werden.

Als wassermischbare organische Lösungsmittel, die zur Durchführung des erfindungsgemässen Herstellungsverfahrens geeignet sind, kommen insbesondere wasserlösliche Alkanole, nämlich solche mit 1 bis 4 C-Atomen, wie Methanol, Ethanol, Propanol, Isopropanol, n-, sec- und iso-Butanol, vorzugsweise aber tert. Butanol, in Betracht.

Der Wassergehalt der hierbei als Lösungsmittel eingesetzten niederen Alkanole sollte 6 Gew.% nicht überschreiten, da sonst eine Klumpenbildung bei der Polymerisation auftreten kann. Vorzugsweise wird bei einem Wassergehalt von 0–3 Gew.% gearbeitet.

Die Menge des einzusetzenden Lösungsmittels richtet sich bis zu einem gewissen Grad nach der Art der eingesetzten Comonomeren.

In der Regel werden pro 100 g Gesamtmonomere 200 bis 1000 g des Lösungsmittels eingesetzt.

Bei der Durchführung der Polymerisation in umgekehrter Emulsion wird die wässrige Monomerenlösung in bekannter Weise in einem mit Wasser nicht mischbaren organischen Lösungsmittel wie Cyclohexan, Toluol, Xylol, Heptan oder hochsiedenden Benzinfraktionen unter Zusatz von 0,5–8 Gew.%, vorzugsweise 1–4 Gew.%, bekannter Emulgatoren vom W/O-Typ emulgiert und mit üblichen radikalbildenden Initiatoren polymerisiert.

Das Prinzip der inversen Emulsionspolymerisation ist aus der U.S. Patentschrift 3 284 393 bekannt. Bei diesem Verfahren werden wasserlösliche Monomere oder Mischungen davon in der Wärme zu hochmolekularen Copolymerisaten polymerisiert, indem man zunächst die Monomeren oder wässrige Lösungen davon, unter Zusatz von Wasser-in-Öl-Emulgatoren in einem mit Wasser nicht mischbaren, die zusammenhängende Phase bildenden organischen Lösungsmittel emulgiert und diese Emulsion in Gegenwart von radikalischen Initiatoren erwärmt. Die einzusetzenden Comonomeren können als solche in dem mit Wasser nicht mischbaren organischen Lösungsmittel emulgiert werden, oder sie können in Form einer wässrigen Lösung, die zwischen 100 und 5 Gewichtsprozent Comonomere und 0 bis 95 Gewichtsprozent Wasser enthält, eingesetzt werden, wobei die Zusammensetzung der wässrigen Lösung eine Frage der Löslichkeit der Comonomeren in Wasser und der vorgesehenen Polymerisationstemperatur ist. Das Verhältnis zwischen Wasser und der Monomerenphase ist in weiten Grenzen variabel und liegt in der Regel bei 70 : 30 bis 30 : 70.

Um die Monomerenphase in dem mit Wasser nicht mischbaren organischen Lösungsmittel zu einer Wasser-in-Öl-Emulsion zu emulgieren, werden den Gemischen 0,1 bis 10 Gewichtsprozent, bezogen auf die Ölphase, eines Wasser-in-Öl-Emulgators, zugesetzt. Vorzugsweise werden solche Emulgatoren verwendet, die einen relativ niedrigen HLB-Wert aufweisen. Als Ölphase kann im Prinzip jede inerte wasserunlösliche Flüssigkeit, d.h. im Prinzip jedes hydrophobe organische Lösungsmittel, eingesetzt werden. Im allgemeinen verwendet man im Rahmen der vorliegenden Erfindung Kohlenwasserstoffe, deren Siedepunkt im Bereich von 120 bis 350 °C liegt. Diese Kohlenwasserstoffe können gesättigte, lineare oder verzweigte Paraffin-Kohlenwasserstoffe, wie sie in Erdölfraktionen vorwiegend vorliegen, sein, wobei diese auch die üblichen Anteile von Naphthenkohlenwasserstoffen enthalten können. Es

können aber auch aromatische Kohlenwasserstoffe, wie beispielsweise Toluol oder Xylol, sowie die Gemische der oben genannten Kohlenwasserstoffe als Ölphase eingesetzt werden. Vorzugsweise verwendet man ein Gemisch aus gesättigten Normal- und Iso-Paraffinkohlenwasserstoffen, das bis zu 20 Gewichtsprozent Naphthene enthält.

Eine detaillierte Beschreibung des Verfahrens findet sich beispielsweise in der deutschen Patentschrift 1 089 173 und in den US-Patentschriften 3 284 393 und 3 624 019.

Vernetzte Copolymerisate mit besonders hohem Polymerisationsgrad in den Grundketten werden erhalten, wenn man die Polymerisation in wässriger Lösung nach dem Verfahren der sogenannten Gelpolymerisation durchführt. Dabei werden 15- bis 60%ige wässrige Lösungen der Comonomeren mit bekannten geeigneten Katalysatorsystemen ohne mechanische Durchmischung unter Ausnutzung des Trommsdorff-Norrisch-Effekts (Bios Final Rep. 363, 22; Makromol. Chem. 1, 169 (1947)) polymerisiert.

Die Polymerisationsreaktion wird im Temperaturbereich zwischen −20 °C und 150 °C, bevorzugt zwischen 5 und 90 °C, durchgeführt, wobei sowohl unter Normaldruck als auch unter erhöhtem Druck gearbeitet werden kann. In der Regel wird die Polymerisation in einer Schutzgasatmosphäre, vorzugsweise unter Stickstoff, ausgeführt.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert. Butyl-hydroperoxid, Methylethyl-keton-peroxid, Cumol-hydroperoxid, Azoverbindungen wie Azo-di-iso-butyro-nitril oder 2′-Azo-bis-(2-amidinopropan)-dihydrochlorid

$$HN{=}\underset{\mid \atop NH_2}{C}{-}C(CH_3)_2{-}N{=}N{-}C(CH_3)_2{-}\underset{\mid \atop NH_2}{C}{=}NH \cdot 2\,HCl$$

sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren enthalten, wie z.B. Mannichaddukte aus Sulfinsäure, Aldehyden und Amino-Verbindungen, wie sie in der deutschen Patentschrift 1 301 566 beschrieben sind. Pro 100 g Gesamtmonomeren werden in der Regel 0,03 bis 2 g des Polymerisationsinitiators eingesetzt.

Es ist weiterhin bekannt, den Polymerisationsansätzen kleine Mengen von sogenannten Moderatoren zuzusetzen, die den Verlauf der Reaktion dadurch harmonisieren, dass sie das Reaktionsgeschwindigkeits-Zeitdiagramm abflachen. Sie führen damit zu einer Verbesserung der Reproduzierbarkeit der Reaktion und ermöglichen damit, einheitliche Produkte mit äusserst geringen Qualitätsabweichungen herzustellen. Beispiele für geeignete Moderatoren dieses Typs sind Nitrilo-trispropionylamid oder Monoalkylamine, Dialkylamine oder Trialkylamine, wie z.B. Dibutylamin. Auch bei der Herstellung der erfindungsgemässen Copolymerisate können solche Moderatoren mit Vorteil verwendet werden.

Weiterhin können den Polymerisationsansätzen sogenannte Regulatoren zugesetzt werden; das sind solche Verbindungen, die das Molekulargewicht der hergestellten Polymerisate beeinflussen. Brauchbare bekannte Regulatoren sind z.B. Alkohole, wie Methanol, Ethanol, Propanol, Isopropanol, n-Butanol, sec.-Butanol und Amylalkohole, Alkylmercaptane, wie z.B. Dodecylmercaptan und tert.-Dodecylmercaptan, Isooctylthioglycolat und einige Halogenverbindungen wie z.B. Tetrachlorkohlenstoff, Chloroform und Methylenchlorid.

Durch mehrstündiges Nachheizen der nach dem Verfahren der Gelpolymerisation erhaltenen Polymerisatgele im Temperaturbereich von 50–130 °C, vorzugsweise 70–100 °C, können die Qualitätseigenschaften der Polymerisate noch verbessert werden.

Die auf diesem Wege hergestellten, in Form wässriger Gallerten vorliegenden erfindungsgemässen, vernetzten Copolymerisate können nach mechanischer Zerkleinerung getrocknet und in fester Form erhalten werden.

Wie bereits weiter oben ausgeführt, eignen sich die erfindungsgemässen neuen Polymeren beispielsweise vorzüglich zur Herstellung von säurelöslichen Überzügen und Verkapselungsmaterialien sowie als Adsorbentien für wässrige Flüssigkeiten. Ausserdem eignen sie sich vorzüglich als Hilfsmittel zur Verminderung des Wasserverlustes von Zementschlämmen, insbesondere solchen für die Zementierung von Tiefbohrungen und Tunnelbauten, sowie als Hilfsmittel für das Färben von Textilien, insbesondere nach Klotzfärbeverfahren, wo sie zu einer drastischen Erhöhung der Flottenaufnahme führen, und Färbungen hoher Farbsättigung und Egalität liefern.

Die folgenden Ausführungsbeispiele veranschaulichen die Herstellung und Anwendung der erfindungsgemässen vernetzten Copolymerisate.

Soweit in den Beispielen der Vernetzer der Formel I in Form eines Rohprodukts des Verfahrens gemäss der EP-A-32 663 eingesetzt wurde, ist er als «Vinylphosphonsäureanhydrid» (VPA) bezeichnet worden.

Im übrigen wurden die folgenden Abkürzungen für die in den Beispielen und Tabellenbeispielen eingesetzen Monomeren benutzt:

| | |
|---|---|
| AM | = Acrylamid |
| AIBS | = 2-Acrylamido-2-methyl-propan-1-sulfonsäure |
| VPS | = Vinylphosphonsäure |
| VPE | = Vinylphosphonsäure-ethylester |
| VIMA | = Vinyl-methyl-acetamid |
| VIFA | = Vinylformamid |
| AS | = Acrylsäure |
| VIPY | = Vinylpyrrolidon |
| MAS | = Methacrylsäure |
| VSSNa | = Vinylsulfonsäure-natriumsalz |
| StyrolSS | = Styrolsulfonsäure |

Beispiel 1
(Emulsionspolymerisation)

7,2 g ®Arkopal N 100 (nichtionischer Emulgator auf der Basis eines oxethylierten Phenolderivats) und 19,4 g ®Span 80 (nichtionischer Emulgator auf Basis eines Zuckeralkohol-Stearats) werden in ®Isopar M (Technisches Gemisch von Isoparaffin mit einem Siedepunkt von ca. 200–240 °C) aufgelöst und die resultierende Lösung in ein 1 l-Reaktionsgefäss eingeschüttet, das mit einem Rührer, Thermometer und einem Stickstoffeinlass versehen ist. Dann wird eine Monomerlösung durch Auflösung von 97,2 g Acrylamid, 9,7 g AIBS, 2,0 g Vinylphosphonsäure (VPS) und 0,5 g einer Verbindung der allgem. Formel Ia, wobei m = 0 und $R^1$ und $R^2$ Wasserstoff ist, in 105 ml Wasser hergestellt. Der pH-Wert der Monomerlösung wird auf 8,5 mit Ammoniak (25%ig) eingestellt. Unter schnellem Rühren wird die wässrige Monomerlösung der organischen Phase zugefügt. Das Reaktionsgefäss wird evakuiert und anschliessend mit Stickstoff gefüllt. Nun wird die Lösung von 0,0275 g Ammoniumpersulfat in 3 ml Wasser dem Gemisch zugefügt und damit die Polymerisation gestartet. Die Reaktion dauert 1,5 Stunden, die Reaktionstemperatur wird zwischen 30 und 40 °C gehalten. Es resultiert eine stabile Emulsion, die unter Verwendung handelsüblicher oberflächenaktiver Mittel in an sich bekannter Weise in Wasser invertiert werden kann, wobei eine hochviskose thixotrope Polymerzubereitung gebildet wird.

Beispiel 2
(Gelpolymerisation)

In einem Polymerisationskolben von 1 l Inhalt, ausgestattet mit Planschliffdeckel, Rührer, Thermometer und Gaseinleitungsrohr wird durch Auflösung von 60 g Acrylamid, 30 g AIBS und 10 g rohem Vinylphosphonsäuremethylester, hergestellt nach dem Verfahren der EP-A-32 663, enthaltend 0,5 g von Verbindungen der Formel

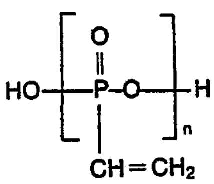

in denen n 2 oder >2 ist in 250 g Wasser eine Monomerlösung hergestellt. Der pH-Wert wird mit Ammoniak (25%ig) auf 8,5 eingestellt. Unter Rühren und Einleiten von Stickstoff werden nun 1 g einer wässrigen 10%igen Dibutylamin-HCl-Lösung und 0,1 g Ammoniumpersulfat zugegeben. Man lässt unter Stickstoff-Einleitung noch 3 Min. bei erhöhter Drehzahl rühren. Die Stickstoff-Einleitung wird beendet, Einleitungsrohr und Rührer werden hochgezogen. Nach einer Induktionszeit von 30 Min. setzt die Polymerisation ein, wobei die Temperatur von 20 °C auf 78 °C ansteigt und die Lösung in ein formstabiles Gel übergeht. Dieses oder ein daraus durch Trocknung und Mahlung hergestelltes Pulver ist in Wasser quellbar, in 2–25%iger wässriger HCl löslich.

K-Wert: 197,8

Beispiel 3
(Fällungspolymerisation)

In einem Polymerisationskolben von 1 l Inhalt, ausgestattet mit Rührer, Rückflusskühler, Thermometer, Tropftrichter und Gaseinleitungsrohr werden in 440 ml tert.-Butanol, 49,7 g Acrylamid, 7,1 g AIBS, 10,7 g VPE, 3,6 g Methacrylsäure (MAS) und 1 g Vinylphosphonsäureanhydrid gelöst. Unter Rühren und Einleiten von Stickstoff wird die Monomerlösung auf 50 °C angeheizt und 1 g Azoisobutyronitril, gelöst in 5 ml DMF, zugetropft. Nach einer Induktionszeit von 30 Min. setzt die Polymerisation ein, die Reaktionstemperatur steigt auf 68 °C und das Polymerisat fällt aus. Es wird noch 2 Std. bei 80 °C nachgeheizt. Das Copolymerisat kann durch Absaugen und Trocknen isoliert werden. Das Lösungsmittel kann jedoch auch direkt unter vermindertem Druck abdestilliert werden. Man erhält das Polymere in Form eines weissen leichten Pulvers, das in Wasser quellbar ist, sich aber in 10%iger wässriger HCl auflöst und einen K-Wert von 108,1 hat.

Das oben eingesetzte Vinylphosphonsäureanhydrid wird in bekannter Weise erhalten durch Hydrolyse von 2 mol Vinyl-phosphonsäure-dichlorid mit 3 mol Wasser.

Ein ebenfalls in Wasser quellbares, aber in 10%iger wässriger HCl deutlich langsamer lösliches Produkt wird erhalten, wenn man in analoger Weise 44,0 g Acrylamid, 7,0 g AIBS, 10 g VPE, 4 g Methacrylsäure und 4 g Vinylphosphonsäureanhydrid einsetzt.

Beispiel 4
(Gelpolymerisation)

In einem Polymerisationskolben von 1 l Inhalt, ausgestattet mit Planschliffdeckel, Rührer, Thermometer und Gaseinleitungsrohr wird durch Auflösung von 65 g Acrylamid, 4,5 g AIBS und 0,5 g eines Vinylphosphonsäure-methyl-ester-anhydrids der Formel Ib

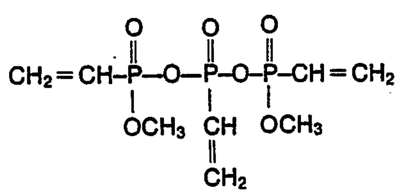

(Ib)

in 250 g Wasser eine Monomerlösung hergestellt. Der pH-Wert wird mit Ammoniak (25%ig) auf 8,5 eingestellt. Unter Rühren und Einleiten von Stickstoff werden nun 1 g einer wässrigen 10%igen Dibutylamin-HCl-Lösung und 0,1 g Ammoniumpersulfat zugegeben. Man lässt unter Stickstoff-Einleitung noch 3 Min. bei erhöhter Drehzahl rühren. Die Stickstoff-Einleitung wird beendet, Einleitungsrohr und Rührer werden hochgezogen. Nach einer Induktionszeit von 30 Min. setzt die Polymerisation ein, wobei die Temperatur von 20 °C auf 78 °C ansteigt und ein formstabiles Gel entsteht.

Durch Trocknen des zerkleinerten Gels kann ein Produkt erhalten werden, das in Wasser stark quillt, in 2%iger Salzsäure löslich ist.

Der oben als Vernetzer eingesetzte Vinylphosphonsäure-methylester-anhydrid wurde durch Umsetzung von 2 Mol Vinylphosphonsäure-monomethylester mit 1 Mol Vinylphosphonsäure-dichlorid bei 80 °C in an sich bekannter Weise hergestellt.

Zu einem Produkt mit praktisch den gleichen Eigenschaften gelangt man, wenn man anstelle des oben eingesetzten Vernetzers der Formel Ib den analog herstellbaren entsprechenden Dibutylester der Formel Ic

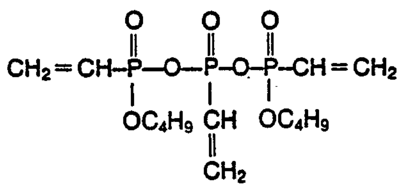
(Ic)

einsetzt.

Beispiel 5
(Fällungspolymerisation)

In einem Polymerisationskolben von 1 l Inhalt, ausgestattet mit Rührer, Rückflusskühler, Thermometer, Tropftrichter und Gaseinleitungsrohr werden in 440 ml tert.-Butanol 49,7 g Acrylamid, 7,1 g AIBS, 10,7 g VPE, 3,6 g Methacrylsäure (MAS) und 1 g eines Vinylphosphonsäure-anhydrids der Formel Id

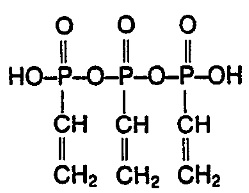
(Id)

(entsprechend einer Verbindung der Formel Ia mit m = 1 und $R^1 = R^2$ = Wasserstoff) gelöst. Unter Rühren und Einleiten von Stickstoff wird die Monomerlösung auf 50 °C angeheizt und 1 g Azoisobutyronitril, gelöst in 5 ml DMF, zugetropft. Nach einer Induktionszeit von 30 Min. setzt die Polymerisation ein, die Reaktionstemperatur steigt auf 68 °C und das Polymerisat fällt aus. Es wird noch 2 Std. bei 80 °C nachgeheizt. Das Copolymerisat kann durch Absaugen und Trocknen isoliert werden. Es kann jedoch auch das Lösungsmittel direkt unter vermindertem Druck abdestilliert werden. Man erhält das Polymere in Form eines weissen leichten Pulvers, das in Wasser quellbar ist, sich aber in 10%iger wässeriger HCl auflöst und einen K-Wert von 98,0 hat.

Beispiel 6
(Gelpolymerisation)

In einem Polymerisationskolben von 1 l Inhalt, ausgestattet mit Planschliffdeckel, Rührer, Thermometer und Gaseinleitungsrohr wird durch Auflösung von 65 g Acrylamid, 4,5 g AIBS und 1,5 g eines Vinylphosphonsäure-Anhydrids der Formel Ie

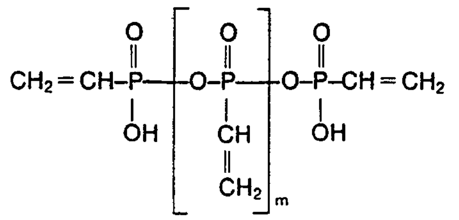
(Ie),

worin m im statistischen Mittel 4,5 ist, in 250 g Wasser eine Monomerlösung hergestellt. Der pH-Wert wird mit Ammoniak (25%ig) auf 8,5 eingestellt. Unter Rühren und Einleiten von Stickstoff werden nun 1 g einer wässrigen 10%igen Dibutylamin-HCl-Lösung und 0,1 g Ammoniumpersulfat zugegeben. Man lässt unter Stickstoff-Einleitung noch 3 Min. bei erhöhter Drehzahl rühren. Die Stickstoff-Einleitung wird beendet, Einleitungsrohr und Rührer werden hochgezogen. Nach einer Induktionszeit von 30 Min. setzt die Polymerisation ein, wobei die Temperatur von 20 °C auf 78 °C ansteigt und ein formstabiles Gel entsteht.

Durch Trocknen des zerkleinerten Gels kann ein Produkt erhalten werden, das in Wasser stark quillt, in 2%iger Salzsäure löslich ist.

Das oben als Vernetzer eingesetzte Vinylphosphonsäure-anhydrid wurde durch Umsetzung von 6 Mol Vinylphosphonsäure-dichlorid mit 7 mol Wasser in an sich bekannter Weise hergestellt.

Beispiel 7
(Fällungspolymerisation)

In einem Polymerisationskolben von 1 l Inhalt, ausgestattet mit Rührer, Rückflusskühler, Thermometer, Tropftrichter und Gaseinleitungsrohr werden in 440 ml tert.-Butanol 49,7 g Acrylamid, 7,1 g AIBS, 10,7 g VPE, 3,6 g Methacrylsäure (MAS), 0,4 g eines Vinylphosphonsäure-anhydrids der Formel Id

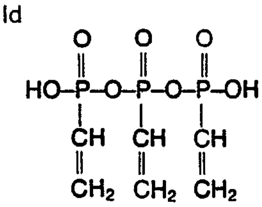
(Id)

0,4 g eines Vinylphosphonsäure-ester-anhydrids der obigen Formel Ib und 0,2 g eines Vinylphosphonsäure-ester-anhydrids der obigen Formel Ic gelöst. Unter Rühren und Einleiten von Stickstoff wird die Monomerlösung auf 50 °C angeheizt und 1 g Azoisobutyronitril, gelöst in 5 ml DMF, zugetropft. Nach einer Induktionszeit von 30 Min. setzt die Polymerisation ein, die Reaktionstemperatur steigt auf 68 °C und das Polymerisat fällt aus. Es wird noch 2 Std. bei 80 °C nachgeheizt. Das Copolymerisat kann durch Absaugen und Trocknen isoliert werden. Es kann jedoch auch das Lösungsmittel direkt unter vermindertem Druck abdestilliert werden. Man erhält das Polymere in Form eines weissen leichten Pulvers, das in Wasser quellbar ist, sich aber in 10%iger wässriger HCl auflöst und einen K-Wert von 110 hat.

Gemäss dieser Verfahrensweisen können auch die Copolymerisate der folgenden Tabelle hergestellt werden.

| | VPA | AM | AIBS | VPS | VPE | VIMA | VIFA | AS | VIPY | MAS | VSSNa | Styrol SS | Viskosität [N·s·m$^{-2}$ |
|---|---|---|---|---|---|---|---|---|---|---|---|---|---|
| 1 | 0,2 | 50 | 20 | 4,8 | 5,0 | 10 | | 5 | | 5 | | | 0,048 |
| 2 | 1,0 | 40 | 9 | 5,0 | 10 | 10 | | 15 | 10 | | | | |
| 3 | 0,5 | 45 | 15 | 24,5 | | 10 | | 5 | | | | | |
| 4 | 1,0 | 25 | 10 | 30 | 9 | | 5 | 20 | | | | | |
| 5 | 0,3 | 5 | 20 | 74,7 | | | | | | | 8 | | |
| 6 | 2,0 | 50 | 9 | 31 | | | | | | | | | 0,029 |
| 7 | 0,1 | 40 | 9 | 20,9 | 10 | | 10 | | 5 | | | 5 | |
| 8 | 0,2 | 50 | 10 | 10 | 24,8 | | | 15 | 10 | | | | |
| 9 | 0,3 | 50 | 10 | 10 | 24,8 | | | | 10 | 15 | | | |
| 10 | 0,5 | 30 | 20 | 20 | 2,5 | 7 | 10 | | | 10 | | | 0,017 |
| 11 | 2,0 | 25 | 15 | 8 | | 30 | | 20 | | | | | |
| 12 | 2,0 | 30 | 20 | 25 | 3,0 | | 12 | | | | | 10 | |
| 13 | 1,0 | 10 | 9 | 10 | | 70 | | | | | | | |
| 14 | 3,0 | 5 | 80 | 2 | | | | 10 | | | | | |
| 15 | 0,5 | 90 | 5 | 2,5 | 2,0 | | | | | | | | |
| 16 | 0,2 | 95 | 2 | 2,8 | | | | | | | | | 0,053 |
| 17 | 0,1 | 98 | 1 | 0,9 | | | | | | | | | 0,084 |
| 18 | 0,3 | 85 | 5 | 1,7 | 3,0 | | 5 | | | | | | 0,107 |
| 19 | 0,5 | 90 | 7,5 | 1,0 | | | | | | | 1,0 | | |
| 20 | 0,5 | 50 | 30 | 5 | 5 | | 4,5 | 5 | | | | | |
| 21 | 0,1 | 60,9 | 20 | 9 | 2 | | | | 8 | | | | |
| 22 | 0,3 | 40 | 15 | 5 | 4,7 | 15 | | | 10 | 10 | | | |
| 23 | 0,3 | 80 | 10 | 4,7 | 5,0 | | | | | | | | 0,048 |
| 24 | 1,0 | 70 | 20 | 5,0 | 4,0 | | | | | | | | 0,205 |
| 25 | 2,0 | 70 | 20 | 5,0 | 3,0 | | | | | | | | |
| 26 | 5,0 | 70 | 20 | 5,0 | | | | | | | | | |
| 27 | 10 | 70 | 20 | | | | | | | | | | 0,108 |
| 28 | 15 | 70 | 10 | 2,0 | 3,0 | | | | | | | | |
| 29 | 20 | 70 | 10 | | | | | | | | | | |
| 30 | 30 | 60 | 5 | 20 | 3,0 | | | | | | | | 0,0132 |
| 31 | 0,4 | 60 | 35 | 2,6 | 2,0 | | | | | | | | |
| 32 | 0,2 | 60 | 36 | 2,8 | 1,0 | | | | | | | | |
| 33 | 0,1 | 60 | 36 | 2,9 | 1,0 | | | | | | | | |
| 34 | 0,5 | 30 | 60 | 2,5 | 1,0 | | 6,0 | | | | | | 0,318 |
| 35 | 0,1 | 30 | 60 | 2,9 | 1,0 | 6,0 | | | | | | | |
| 36 | 0,5 | 30 | 50 | 2,5 | 1,0 | 10 | | 6 | | | | | |
| 37 | 1,5 | 50 | 20 | 2,5 | 1,0 | | | | 20 | | 5 | | |
| 38 | 2,5 | 60 | 10 | 2,5 | | 10 | | | | 10 | | 5 | |

Anwendungstechnisches Beispiel 1

Wasserverlust aus Zementschlämme

Zementschlämme, die zur Zementierung von Gesteinshohlräumen, wie z. B. Bohrlöchern für die Erdölgewinnung oder auch Tunnelausbauten eingesetzt werden, stehen in unmittelbarer Berührung mit porösen Gesteinsformationen, was bei normalen Zementmischungen zu starker Wasserabgabe und damit zu unvollständiger und ungleichmässiger Aushärtung führt.

Zur Gewährleistung einer technisch einwandfreien Zementierung von Bohrlöchern und Tunnelwänden wird ein möglichst geringer Wasserverlust der eingesetzten Zementschlämme angestrebt.

Der Wasserverlust kann im Labor mit einer Filterpresse nach APJ·Code 29 gemessen werden, wobei die Filterfläche 45,8 ± 0,7 cm$^2$, der Überdruck 7 ± 0,7 bar beträgt. Die Messung des von der Zementschlämme abgegebenen Wasservolumens erfolgt nach einer Filtrationsdauer von 30 Minuten. Von besonderer Bedeutung ist auch die Elektrolytstabilität der Retardierwirkung des Polymerzusatzes, da in geologischen Formationen häufig in Gegenwart von wasserlöslichen Salzen gearbeitet werden muss und auch unter diesen Bedingungen der Wasserverlust möglichst gering sein soll. Für die Laboruntersuchung wird daher als Anmachwasser für den Zement eine gesättigte NaCl-Lösung eingesetzt.

Zur Prüfung werden 500 g Zement Klasse G mit 250 g gesättigter Kochsalzlösung homogen angeteigt und dann 2,5 g eines erfindungsgemässen Copolymerisats der folgenden Tabelle zugesetzt und gleichmässig verrührt. Dann wird sofort die Filtrationsprobe nach APJ Code 29 durchgeführt. Blindprobe, Zementteig ohne erfindungsgemässen Zusatz:

Wasserverlust = 86 ml.

| Polymerisat Beispiel Nr. | Wasserverlust APJ Code 29 (ml) |
|---|---|
| 14 | 12,3 |
| 35 | 15 |
| 36 | 16,8 |

Anwendungstechnisches Beispiel 2
Klotzfärbeverfahren

Bei diesem Färbeverfahren ist es von Bedeutung, eine hohe und gleichmässige Flottenaufnahme zu erzielen. Ein Baumwoll-Wirkmaterial wird auf einem Foulard für Trikot bei Raumtemperatur mit einer Flotte geklotzt, die im Liter 55 g des Reaktivfarbstoffes Reactive Black 5 (C.I. No. 20505), 8 g eines handelsüblichen Netzmittels und 30 g einer 5gew%igen thixotropen wässrigen Paste des Copolymeren gem. Beispiel 17 enthält. Das erforderliche Fixieralkali wird in das Chassis eindosiert. Die Flottenaufnahme beträgt 145%. Nach dem Verweilen der geklotzten Ware unter langsamer Rotation über Nacht und der üblichen Nachbehandlung erhält man auf dem Baumwolltrikot eine ungewöhnlich volle und hervorragend egale Schwarzfärbung.

Ohne Polymerzusatz beträgt die Flottenaufnahme 103% bei gleichem Abquetschdruck. Wird das oben eingesetzte erfindungsgemässe Copolymerisat des Beispiels 17 durch die gleiche Menge der Copolymerisate der Beispiele 19 oder 21 ersetzt, so erhält man bei gleichem Abquetschdruck Flottenaufnahmen von 161% bzw. 138%.

Anwendungstechnisches Beispiel 3
Absorption wässriger Flüssigkeiten

Zur Prüfung auf Absorptionsvermögen für wässrige Flüssigkeiten wird 1 g pulverförmiges Polymer in 500 ml Wasser eingerührt. Nach 15 Minuten wird die Mischung über ein Filtertuch abfiltriert und, sobald kein Wasser mehr abtropft, das Gewicht der zurückbleibenden gequollenen Gelteilchen bestimmt.

Unter Verwendung der in der Tabelle angegebenen erfindungsgemässen Copolymerisate wurden folgende Ergebnisse erhalten.

| Copolymerisat des Beispiels Nr. | Gewicht der gequollenen Teilchen (g) |
|---|---|
| 15 | 65 |
| 23 | 85 |
| 24 | 60 |
| 25 | 51 |
| 26 | 34 |
| 30 | 22 |

**Patentansprüche für die Vertragsstaaten BE, CH, DE, FR, GB, IT, LI, NL, SE**

1. Vernetztes hydrophiles Copolymerisat, dadurch gekennzeichnet, dass es 0,01 bis 30 Gew.% Brückenglieder der Formel I

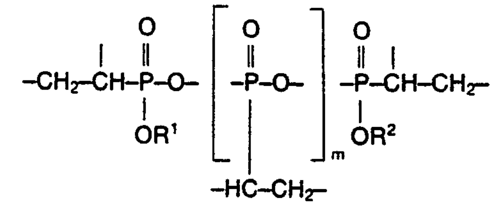

(I)

worin $R^1$ und $R^2$ unabhängig voneinander Wasserstoff oder Alkyl mit 1 bis 4 C-Atomen bedeuten und m Werte von 0 bis 6 besitzt, und 99,99 bis 70 Gew.% Grundkettenbausteine der Formel III

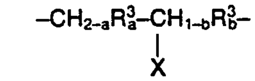

(III)

worin

$R^3$ Wasserstoff oder Methyl ist, a und b jeweils die Werte 0 oder 1 haben und die Summe a + b 0 oder 1 ist und X die Karbonamidgruppe $-CONH_2$; eine Gruppe der Formel IV

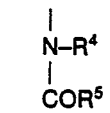

(IV)

worin $R^4$ und $R^5$ unabhängig voneinander für Wasserstoff, Methyl oder Ethyl oder gemeinsam für Trimethylen oder Pentamethylen stehen; Carboxyl oder dessen Salz mit einem Kation $M^{\oplus}$; Alkoxycarbonyl mit 1 bis 6 Kohlenstoffatomen im Alkylteil; Hydroxyalkoxycarbonyl mit 1 bis 3 Kohlenstoffatomen im Hydroxyalkylteil; N-Methylolcarbonamid der Formel $HOCH_2NH-CO-$, dessen Methylolgruppe gegebenenfalls mit Alkanolen mit 1 bis 4 Kohlenstoffatomen verethert sein kann; Alkanoylamino mit 1 bis 4 Kohlenstoffatomen, das gegebenenfalls durch Methylol oder Alkyl mit 1 bis 4 Kohlenstoffatomen N-substituiert sein kann; Cyan; gegebenenfalls durch Alkyl mit 1 bis 4 Kohlenstoffatomen, Alkoxy mit 1 oder 2 Kohlenstoffatomen, Halogen, Trifluormethyl oder Nitro substituiertes Phenyl oder Benzyl; Imidazolyl-(1); die Sulfonsäuregruppe; Sulfoalkylamido-carbonyl mit 1 bis 4 Kohlenstoffatomen im Alkylrest; die Phosphonsäuregruppe, wobei die Sulfonsäure- und Phosphonsäuregruppen auch in Form ihrer Salze mit einem Kation $M^{\oplus}$ vorliegen können; die Phosphonsäureestergruppe der Formel V

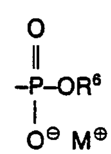

(V)

worin $R^6$ Alkyl mit 1 bis 4 C-Atomen ist; einen Rest der Formel VI

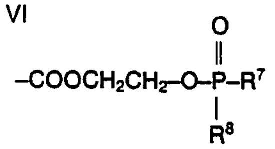

(VI)

worin $R^7$ und $R^8$ gleich oder verschieden sind und für Alkyl mit 1 bis 7 Kohlenstoffatomen stehen; einen Rest der Formel VII

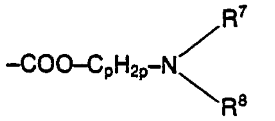

(VII)

worin $R^7$ und $R^8$ die oben angegebenen Bedeutungen haben und p für eine Zahl von 1 bis 4 steht; oder einen Rest der Formel VIII

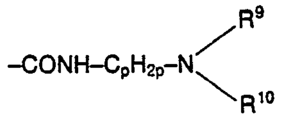

(VIII)

worin $R^9$ und $R^{10}$ gleich oder verschieden sind und für Alkyl mit 1 bis 4 Kohlenstoffatomen stehen und p für eine Zahl von 1 bis 4 steht; sowie die den Formeln VII und VIII entsprechenden quaternisierten Gruppen bedeuten, enthält, wobei sich das Kation $M^{\oplus}$ von einer wasserlöslichen Base ableitet, deren Stärke ausreicht, die genannten Sulfonsäure- bzw. Carboxylgruppen zu neutralisieren und die deren Hydrophilie nicht beeinträchtigt.

2. Vernetztes Copolymerisat gemäss Anspruch 1, dadurch gekennzeichnet, dass in den Brückengliedern der Formel I $R^1$ und $R^2$ unabhängig voneinander Wasserstoff oder Alkyl mit 1 oder 2 Kohlenstoffatomen bedeuten.

3. Vernetztes Copolymerisat gemäss einem oder beiden der Ansprüche 1 und 2, dadurch gekennzeichnet, dass in den Grundkettenbausteinen der Formel III das für $R^4$ und/oder $R^5$ stehende Alkoxycarbonyl 1 bis 3 Kohlenstoffatome im Alkylteil und die für $R^6$, $R^7$ und/oder $R^8$ und $R^9$ und/oder $R^{10}$ stehenden Alkylreste 1 oder 2 Kohlenstoffatome enthalten.

4. Vernetztes Copolymerisat gemäss einem oder mehreren der Ansprüche 1 bis 3, dadurch gekennzeichnet, dass die Grundketten aus Bausteinen der Formel IX

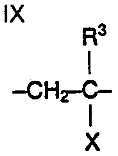

(IX)

worin $R^3$ Wasserstoff oder Methyl und X die Karbonamidgruppe $-CONH_2$; eine Gruppe der Formel IV

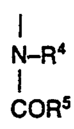

(IV)

worin $R^4$ und $R^5$ unabhängig voneinander für Wasserstoff, Methyl oder Ethyl oder gemeinsam für Trimethylen oder Pentamethylen stehen; Carboxyl oder dessen Alkali- oder Ammoniumsalze, Hydroxyalkoxycarbonyl mit 2 oder 3, vorzugsweise 2, C-Atomen im Hydroxyalkylteil; die Sulfonsäuregruppe; Sulfoalkylamidocarbonyl mit 1 bis 4 C-Atomen im Alkylrest, vorzugsweise eine Gruppe der Formel

$$CO{-}NH{-}C(CH_3)_2{-}CH_2{-}SO_3^{\ominus}M^{\oplus}$$

die Phosphonsäuregruppe, wobei die Sulfonsäure- und Phosphonsäuregruppen auch in Form ihrer Alkali- oder Ammoniumsalze vorliegen können; und die Phosphonsäureestergruppe der Formel V

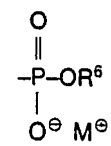

(V)

worin $R^6$ Alkyl mit 1 bis 4, vorzugsweise 1 oder 2, C-Atomen ist, bedeutet, aufgebaut sind.

5. Vernetztes Copolymerisat gemäss einem oder mehreren der Ansprüche 1 bis 4, dadurch gekennzeichnet, dass es 0,01 bis 6 Gew.%, insbesondere 0,05 bis 2 Gew.%, Brückenglieder und 99,99 bis 94 Gew.%, insbesondere 99,95 bis 98 Gew.%, Grundkettenbausteine enthält.

6. Verfahren zur Herstellung von vernetzten Copolymerisaten gemäss einem oder mehreren der Ansprüche 1 bis 5, dadurch gekennzeichnet, dass olefinisch ungesättigte Grundkettenbausteine der Formel IIIa

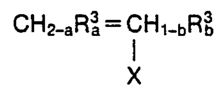

(IIIa)

mit vernetzenden Comonomeren der Formel Ia

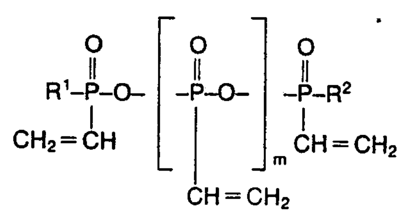

(Ia)

copolymerisiert werden, wobei $R^1$, $R^2$, $R^3$, X, a, b und m die oben definierten Bedeutungen besitzen.

7. Verwendung der vernetzten Copolymerisate gemäss einem oder mehreren der Ansprüche 1 bis 5 zur Herstellung säurelöslicher Überzüge und Verkapselungen.

8. Verwendung der vernetzten Copolymerisate gemäss einem oder mehreren der Ansprüche 1 bis 5 als Färbereihilfsmittel.

9. Verwendung der vernetzten Copolymerisate gemäss einem oder mehreren der Ansprüche 1 bis 5 als Hilfsmittel zur Verminderung des Wasserverlusts von Zementschlämmen.

10. Verwendung der vernetzten Copolymerisate gemäss einem oder mehreren der Ansprüche 1 bis 5 als Absorptionsmittel für wässrige Flüssigkeiten.

**Patentansprüche für den Vertragsstaat AT**

1. Verfahren zur Herstellung von vernetzten hydrophilen Copolymerisaten, dadurch gekennzeichnet, dass 0,01 bis 30 Gew.% vernetzende Comonomere der Formel Ia

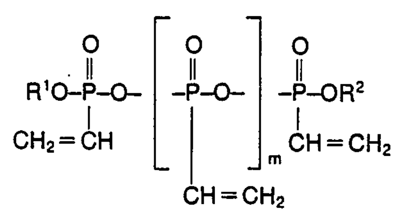

(Ia)

worin $R^1$ und $R^2$ unabhängig voneinander Wasserstoff oder Alkyl mit 1 bis 4 C-Atomen bedeuten und m Werte von 0 bis 6 besitzt, mit 99,99 bis 70 Gew.% olefinisch ungesättigte Grundkettenbausteine der Formel IIIa

$$\begin{array}{c} CH_{2-a}R^3_a = CH_{1-b}R^3_b \\ \quad\quad | \\ \quad\quad X \end{array} \qquad \text{(IIIa)}$$

worin

$R^3$ Wasserstoff oder Methyl ist, a und b jeweils die Werte 0 oder 1 haben und die Summe a+b 0 oder 1 ist und X die Karbonamidgruppe $-CONH_2$; eine Gruppe der Formel IV

$$\begin{array}{l} | \\ N-R^4 \\ | \\ COR^5 \end{array} \qquad \text{(IV)}$$

worin $R^4$ und $R^5$ unabhängig voneinander für Wasserstoff, Methyl oder Ethyl oder gemeinsam für Trimethylen oder Pentamethylen stehen; Carboxyl oder dessen Salz mit einem Kation $M^{\oplus}$; Alkoxycarbonyl mit 1 bis 6 Kohlenstoffatomen im Alkylteil; Hydroxyalkoxycarbonyl mit 1 bis 3 Kohlenstoffatomen im Hydroxyalkylteil; N-Methylolcarbonamid der Formel $HOCH_2NH-CO-$, dessen Methylolgruppe gegebenenfalls mit Alkanolen mit 1 bis 4 Kohlenstoffatomen verethert sein kann; Alkanoylamino mit 1 bis 4 Kohlenstoffatomen, das gegebenenfalls durch Methylol oder Alkyl mit 1 bis 4 Kohlenstoffatomen N-substituiert sein kann; Cyan; gegebenenfalls durch Alkyl mit 1 bis 4 Kohlenstoffatomen, Alkoxy mit 1 oder 2 Kohlenstoffatomen, Halogen, Trifluormethyl oder Nitro substituiertes Phenyl oder Benzyl; Imidazolyl-(1); die Sulfonsäuregruppe; Sulfoalkylamidocarbonyl mit 1 bis 4 Kohlenstoffatomen im Alkylrest; die Phosphonsäuregruppe, wobei die Sulfonsäure- und Phosphonsäuregruppen auch in Form ihrer Salze mit einem Kation $M^{\oplus}$ vorliegen können; die Phosphonsäureestergruppe der Formel V

$$\begin{array}{l} \;\;O \\ \;\;\| \\ -P-OR^6 \\ \;\;| \\ \;\;O^{\ominus}\ M^{\oplus} \end{array} \qquad \text{(V)}$$

worin $R^6$ Alkyl mit 1 bis 4 C-Atomen ist; einen Rest der Formel VI

$$\begin{array}{r} O\;\; \\ \|\;\; \\ -COOCH_2CH_2-O-P-R^7 \\ |\;\;\;\; \\ R^8\;\; \end{array} \qquad \text{(VI)}$$

worin $R^7$ und $R^8$ gleich oder verschieden sind und für Alkyl mit 1 bis 7 Kohlenstoffatomen stehen; einen Rest der Formel VII

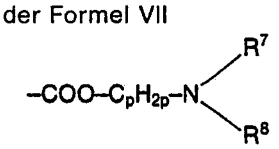 (VII)

worin $R^7$ und $R^8$ die oben angegebenen Bedeutungen haben und p für eine Zahl von 1 bis 4 steht; oder einen Rest der Formel VIII

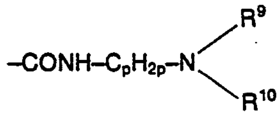 (VIII)

worin $R^9$ und $R^{10}$ gleich oder verschieden sind und für Alkyl mit 1 bis 4 Kohlenstoffatomen stehen und p für eine Zahl von 1 bis 4 steht; sowie die den Formeln VII und VIII entsprechenden quaternisierten Gruppen bedeuten, umgesetzt werden, wobei sich das Kation $M^{\oplus}$ von einer wasserlöslichen Base ableitet, deren Stärke ausreicht, die genannten Sulfonsäure- bzw. Carboxylgruppen zu neutralisieren und die deren Hydrophilie nicht beeinträchtigt.

2. Verfahren zur Herstellung von vernetzten Copolymerisaten gemäss Anspruch 1, dadurch gekennzeichnet, dass in den Comonomeren der Formel Ia $R^1$ und $R^2$ unabhängig voneinander Wasserstoff oder Alkyl mit 1 oder 2 Kohlenstoffatomen bedeuten.

3. Verfahren zur Herstellung von vernetzten Copolymerisaten gemäss einem oder beiden der Ansprüche 1 und 2, dadurch gekennzeichnet, dass in den Comonomeren der Formel IIIa das für $R^4$ und/oder $R^5$ stehende Alkoxycarbonyl 1 bis 3 Kohlenstoffatome im Alkylteil und die für $R^6$, $R^7$ und/oder $R^8$ und $R^9$ und/oder $R^{10}$ stehenden Alkylreste 1 oder 2 Kohlenstoffatome enthalten.

4. Verfahren zur Herstellung von vernetzten Copolymerisaten gemäss einem oder mehreren der Ansprüche 1 bis 3, dadurch gekennzeichnet, dass die Comonomeren der Formel IIIa derart gewählt werden, dass die Grundketten der Copolymerisate aus Bausteinen der Formel IX

$$\begin{array}{c} \quad\; R^3 \\ \quad\; | \\ -CH_2-C- \\ \quad\; | \\ \quad\; X \end{array} \qquad \text{(IX)}$$

worin $R^3$ Wasserstoff oder Methyl und X die Karbonamidgruppe $-CONH_2$; eine Gruppe der Formel IV

$$\begin{array}{l} | \\ N-R^4 \\ | \\ COR^5 \end{array} \qquad \text{(IV)}$$

worin $R^4$ und $R^5$ unabhängig voneinander für Wasserstoff, Methyl oder Ethyl oder gemeinsam für Trimethylen oder Pentamethylen stehen; Carboxyl oder dessen Alkali- oder Ammoniumsalze, Hydroxyalkoxycarbonyl mit 2 oder 3, vorzugsweise 2, C-Atomen im Hydroxyalkylteil; die Sulfonsäuregruppe; Sulfoalkylamidocarbonyl mit 1 bis 4 C-Atomen im Alkylrest, vorzugsweise eine Gruppe der Formel

$$CO-NH-C(CH_3)_2-CH_2-SO_3^{\ominus}M^{\oplus}$$

die Phosphonsäuregruppe, wobei die Sulfonsäure- und Phosphonsäuregruppen auch in Form ihrer Alkali- oder Ammoniumsalze vorliegen können; und die Phosphonsäureestergruppe der Formel V

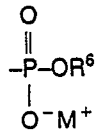 (V)

worin $R^6$ Alkyl mit 1 bis 4, vorzugsweise 1 oder 2, C-Atomen ist, bedeutet, bestehen.

5. Verfahren zur Herstellung von vernetzten Copolymerisaten gemäss einem oder mehreren der Ansprüche 1 bis 4, dadurch gekennzeichnet, dass 0,01 bis 6, insbesondere 0,05 bis 2 Gewichtsteile von Comonomeren der Formel Ia und 99,99 bis 94, insbesondere 99,95 bis 98, Gewichtsteile von Comonomeren der Formel IIIa eingesetzt werden.

6. Verwendung der vernetzten gemäss einem oder mehreren der Ansprüche 1 bis 5 hergestellten Copolymerisate zur Herstellung säurelöslicher Überzüge und Verkapselungen.

7. Verwendung der vernetzten gemäss einem oder mehreren der Ansprüche 1 bis 5 hergestellten Copolymerisate als Färbereihilfsmittel.

8. Verwendung der vernetzten gemäss einem oder mehreren der Ansprüche 1 bis 5 hergestellten Copolymerisate als Hilfsmittel zur Verminderung des Wasserverlustes von Zementschlämmen.

9. Verwendung der vernetzten gemäss einem oder mehreren der Ansprüche 1 bis 5 hergestellten Copolymerisate als Absorptionsmittel für wässrige Flüssigkeiten.

**Claims for the Contracting States: BE, CH, DE, FR, GB, IT, LI, NL, SE**

1. Crosslinked hydrophilic copolymer, characterised in that it contains 0.01 to 30% by weight of bridge members of the formula I

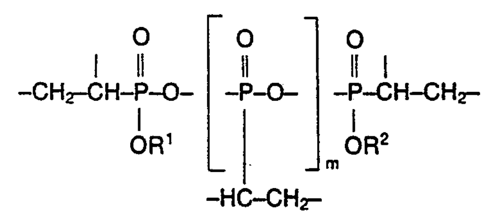 (I)

wherein $R^1$ and $R^2$ independently of one another denote hydrogen or alkyl with 1 to 4 C atoms and m represents a number from 0 to 6, and 99.99 to 70% by weight of basic chain units of the formula III

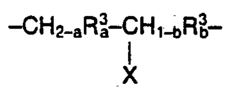 (III)

wherein $R^3$ is hydrogen or methyl, a and b each have the value 0 or 1 and the sum a+b is 0 or 1 and X is the carboxamide group $-CONH_2$; a group of the formula IV

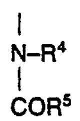 (IV)

wherein $R^4$ and $R^5$ independently of one another represent hydrogen, methyl or ethyl or together represent trimethylene or pentamethylene; carboxyl or a salt thereof with a cation $M^\oplus$; alkoxycarbonyl with 1 to 6 carbon atoms in the alkyl portion; hydroxyalkoxycarbonyl with 1 to 3 carbon atoms in the hydroxyalkyl portion; N-methylolcarboxamide of the formula

$$HOCH_2NH-CO-$$

the methylol group of which can optionally be etherified with alkanols with 1 to 4 carbon atoms; alkanoylamino with 1 to 4 carbon atoms, which can optionally be N-substituted by methylol or alkyl with 1 to 4 carbon atoms; cyano; phenyl or benzyl, optionally substituted by alkyl with 1 to 4 carbon atoms, alkoxy with 1 or 2 carbon atoms, halogen, trifluoromethyl or nitro; imidazol-1-yl; the sulphonic acid group; sulphoalkylamidocarbonyl with 1 to 4 carbon atoms in the alkyl radical; the phosphonic acid group, it also be possible for the sulphonic acid and phosphonic acid groups to be in the form of their salts with a cation $M^\oplus$; the phosphonic acid ester group of the formula V

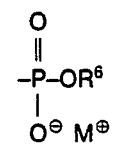 (V)

wherein $R^6$ is alkyl with 1 to 4 C atoms; a radical of the formula VI

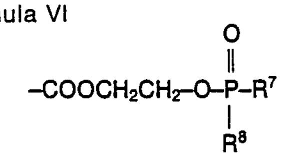 (VI)

wherein $R^7$ and $R^8$ are identical or different and represent alkyl with 1 to 7 carbon atoms; a radical of the formula VII

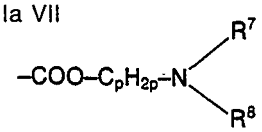 (VII)

wherein $R^7$ and $R^8$ have the abovementioned meanings and p represents a number from 1 to 4; or a radical of the formula VIII

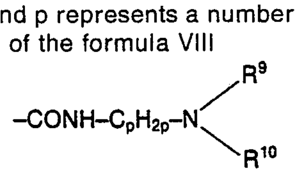 (VIII)

wherein $R^9$ and $R^{10}$ are identical or different and represent alkyl with 1 to 4 carbon atoms and p represents a number from 1 to 4; and the quaternised groups which correspond to the formulae VII and VIII, the cation $M^\oplus$ deriving from a water-soluble base whose strength is sufficient to neutralize the mentioned sulphonic acid and carboxylic groups and which does not impair the hydrophily thereof.

2. Crosslinked copolymer according to Claim 1, characterised in that, in the bridge members of the formula I, $R^1$ and $R^2$ independently of one another denote hydrogen or alkyl with 1 or 2 C atoms.

3. Crosslinked copolymer according to Claim 1 and/or 2, characterised in that in the basic chain units of formula III the alkoxycarbonyl representing $R^4$ and/or $R^5$ contains 1 to 3 carbon atoms in the alkyl portion and the alkyl radicals representing $R^6$, $R^7$ and/or $R^8$ and $R^9$ and/or $R^{10}$ contain 1 or 2 carbon atoms.

4. Crosslinked copolymer according to one or several of Claims 1 to 3, characterised in that the basic chains consist of units of the formula IX

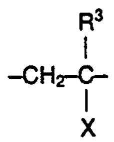

(IX)

wherein $R^3$ denotes hydrogen or methyl and X denotes the carboxamide group $-CONH_2$; a group of the formula IV

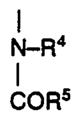

(IV)

wherein $R^4$ and $R^5$ independently of one another represent hydrogen, methyl or ethyl or together represent trimethylene or pentamethylene; carboxyl or alkali metal or ammonium salts thereof or hydroxyalkoxycarbonyl with 2 or 3, preferably 2, carbon atoms in the hydroxyalkyl portion; the sulphonic acid group; sulphoalkylamidocarbonyl with 1 to 4 carbon atoms in the alkyl radical, preferably a group of the formula

$$-CO-NH-C(CH_3)_2-CH_2-SO_3^{\ominus}M^{\oplus},$$

the phosphonic acid group, it also being possible for the sulphonic acid and phosphonic acid groups to be in the form of their alkali metal or ammonium salts; and the phosphonic acid ester group of the formula V

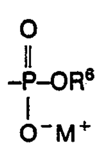

(V)

wherin $R^6$ denotes alkyl with 1 to 4, preferably 1 or 2, C atoms.

5. Crosslinked copolymer according to one or several of Claims 1 to 4, characterised in that it contains 0.01 to 6% by weight, in particular 0.05 to 2% by weight, of bridge members and 99.99 to 94% by weight, in particular 99.95 to 98% by weight, of basic chain units.

6. Process for the preparation of crosslinked copolymers according to one or several of Claims 1 to 5, characterised in that olefinically unsaturated basic chain units of the formula IIIa

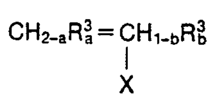

(IIIa)

are copolymerized with crosslinking comonomers of the formula Ia

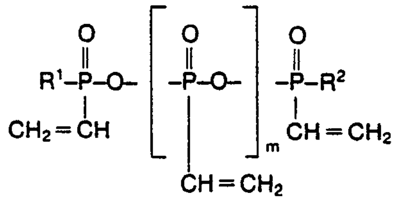

(Ia)

wherein $R^1$, $R^2$, $R^3$, X, a, b and m have the meanings defined above.

7. Use of the crosslinked copolymers according to one or several of Claims 1 to 5 for the preparation of acid-soluble coatings and encapsulations.

8. Use of the crosslinked copolymers according to one or several of Claims 1 to 5 as dyeing auxiliaries.

9. Use of the crosslinked copolymers according to one or several of Claims 1 to 5 as an auxiliary to prevent the water loss of cement sludges.

10. Use of the crosslinked copolymers according to one or several of Claims 1 to 5 as adsorption agents for aqueous liquids.

**Claims for the Contracting State: AT**

1. Process for preparing crosslinked hydrophilic copolymers, characterised in that there are reacted 0.01 to 30% by weight of bridge members of the formula Ia

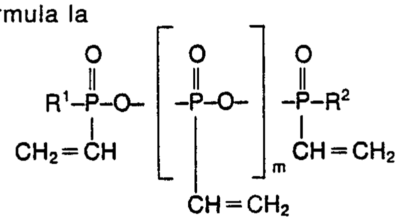

(Ia)

wherein $R^1$ and $R^2$ independently of one another denote hydrogen or alkyl with 1 to 4 C atoms and m represents a number from 0 to 6, and 99.99 to 70% by weight of basic chain units of the formula IIIa

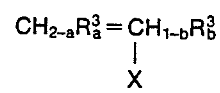

(IIIa)

wherein $R^3$ is hydrogen or methyl, a and b each have the value 0 or 1 and the sum a+b is 0 or 1 and X is the carboxamide group $-CONH_2$; a group of the formula IV

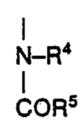

(IV)

wherein $R^4$ and $R^5$ independently of one another represent hydrogen, methyl or ethyl or together represent trimethylene or pentamethylene; carboxyl or a salt thereof with a cation $M^{\oplus}$; alkoxycarbonyl with 1 to 6 carbon atoms in the alkyl portion; hydroxyalkoxycarbonyl with 1 to 3 carbon atoms in the hydroxyalkyl portion; N-methylolcarboxamide of the formula

$$HOCH_2NH-CO-$$

the methylol group of which can optionally be etherified with alkanols with 1 to 4 carbon atoms;

alkanoylamino with 1 to 4 carbon atoms, which can optionally be N-substituted by methylol or alkyl with 1 to 4 carbon atoms; cyano; phenyl or benzyl, optionally substituted by alkyl with 1 to 4 carbon atoms, alkoxy with 1 or 2 carbon atoms, halogen, trifluoromethyl or nitro; imidazol-1-yl; the sulphonic acid group; sulphoalkylamidocarbonyl with 1 to 4 carbon atoms in the alkyl radical; the phosphonic acid group, it also being possible for the sulphonic acid and phosphonic acid groups to be in the form of their salts with a cation $M^{\oplus}$; the phosphonic acid ester group of the formula V

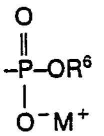
(V)

wherein $R^6$ is alkyl with 1 to 4 C atoms; a radical of the formula VI

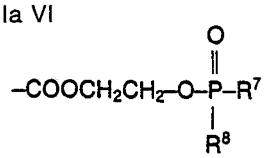
(VI)

wherein $R^7$ and $R^8$ are identical or different and represent alkyl with 1 to 7 carbon atoms; a radical of the formula VII

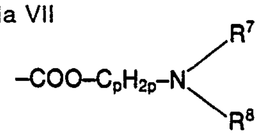
(VII)

wherein $R^7$ and $R^8$ have the abovementioned meanings and p represents a number from 1 to 4; or a radical of the formula VIII

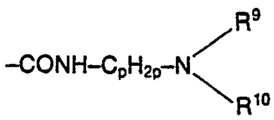
(VIII)

wherein $R^9$ and $R^{10}$ are identical or different and represent alkyl with 1 to 4 carbon atoms and p represents a number from 1 to 4; and the quaternised groups which correspond to the formulae VII and VIII, the cation $M^{\oplus}$ deriving from a water-soluble base whose strength is sufficient to neutralize the mentioned sulphonic acid and carboxylic groups and which does not impair the hydrophily thereof.

2. Process for preparing crosslinked copolymers according to Claim 1, characterised in that, in the comonomers of formula Ia, $R^1$ and $R^2$ independently of one another denote hydrogen or alkyl with 1 or 2 C atoms.

3. Process for preparing crosslinked copolymers according to one or both of Claims 1 and 2, characterised in that in the comonomers of formula IIIa the alkoxycarbonyl representing $R^4$ and/or $R^5$ contains 1 to 3 carbon atoms in the alkyl portion and the alkyl radicals representing $R^6$, $R^7$ and/or $R^8$ and $R^9$ and/or $R^{10}$ contain 1 or 2 carbon atoms.

4. Process for preparing crosslinked copolymers according to one or several of Claims 1 to 3, characterised in that the comonomers of formula IIIa are chosen thus that the basic chains of the copolymers consist of units of the formula IX

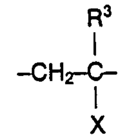
(IX)

wherein $R^3$ denotes hydrogen or methyl and X denotes the carboxamide group $-CONH_2$; a group of the formula IV

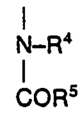
(IV)

wherein $R^4$ and $R^5$ independently of one another represent hydrogen, methyl or ethyl or together represent trimethylene or pentamethylene; carboxyl or alkali metal or ammonium salts thereof or hydroxyalkoxycarbonyl with 2 or 3, preferably 2, carbon atoms in the hydroxyalkyl portion; the sulphonic acid group; sulphoalkylamidocarbonyl with 1 to 4 carbon atoms in the alkyl radical, preferably a group of the formula

$$-CO-NH-C(CH_3)_2-CH_2-SO_3^{\ominus}M^{\oplus},$$

the phosphonic acid group, it also being possible for the sulphonic acid and phosphonic acid groups to be in the form of their alkali metal or ammonium salts; and the phosphonic acid ester group of the formula V

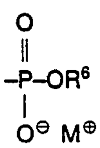
(V)

wherein $R^6$ denotes alkyl with 1 to 4, preferably 1 or 2, C atoms.

5. Process for preparing crosslinked copolymers according to one or several of Claims 1 to 4, characterised in that 0.01 to 6, in particular 0.05 to 2 parts by weight, of comonomers of formula Ia and 99.99 to 94, in particular 99.95 to 98 parts by weight, of comonomers of formula IIIa are employed.

6. Use of the crosslinked copolymers prepared according to one or several of Claims 1 to 5 for the preparation of acid-soluble coatings and encapsulations.

7. Use of the crosslinked copolymers prepared according to one or several of Claims 1 to 5 as dyeing auxiliaries.

8. Use of the crosslinked copolymers prepared according to one or several of Claims 1 to 5 as an auxiliary to prevent the water loss of cement sludges.

9. Use of the crosslinked copolymers prepared according to one or several of Claims 1 to 5 as adsorption agents for aqueous liquids.

**Revendications pour les Etats contractants BE, CH, DE, FR, GB, IT, LI, NL, SE**

1. Copolymère réticulè hydrophile, caractérisé en ce qu'il contient de 0,01 à 30% en poids de groupements de liaison répondant à la formule I:

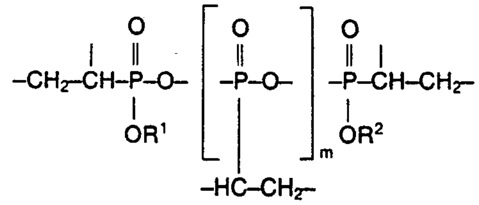
(I)

dans laquelle: $R^1$ et $R^2$ représentent chacun, indépendamment l'un de l'autre, l'hydrogène ou un alkyle contenant de 1 à 4 atomes de carbone et m désigne un nombre de 0 à 6, et de 99,99 à 70% en poids d'éléments constitutifs de chaînes de base qui répondent à la formule III:

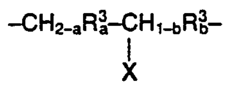
(III)

dans laquelle: $R^3$ représente l'hydrogène ou un méthyle, a et b sont égaux chacun à 0 ou à 1, la somme (a+b) étant égale à 0 ou à 1, et X représente: un radical carbamoyle $-CONH_2$, un radical de formule IV:

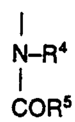
(IV)

dans lequel $R^4$ et $R^5$ représentent chacun, indépendamment l'un de l'autre, l'hydrogène, un méthyle ou un éthyle ou forment ensemble un radical triméthylène ou pentaméthylène, un carboxy ou un sel de celui-ci avec un cation $M^{\oplus}$, un alcoxycarbonyle dont la partie alkyle contient de 1 à 6 atomes de carbone, un hydroxyalcoxycarbonyle dont la partie hydroxyalkyle contient de 1 à 3 atomes de carbone, un radical N-(hydroxyméthyl)-carbamoyle $HOCH_2NH-CO-$ dont le radical hydroxyméthyle est éventuellement éthérifié par un alcanol contenant de 1 à 4 atomes de carbone, un alcanoylamino en $C_1-C_4$ qui porte éventuellement un radical hydroxyméthyle ou un alkyle contenant de 1 à 4 atomes de carbone, un radical cyano, un radical phényle ou benzyle éventuellement porteur d'un alkyle contenant de 1 à 4 atomes de carbone, d'un alcoxy contenant 1 ou 2 atomes de carbone, d'un halogène, d'un trifluorométhyle ou d'un nitro, un radical imidazolyle-1, un radical sulfo, un radical sulfoalkyl-aminocarbonyle dont la partie alkyle contient de 1 à 4 atomes de carbone, un radical phosphono, le radical sulfo et le radical phosphono pouvant également être sous la forme d'un sel avec un cation $M^{\oplus}$, un radical d'ester phosphonique de formule V:

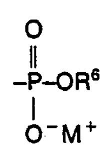
(V)

dans lequel $R^6$ représente un alkyle contenant de 1 à 4 atomes de carbone, un radical de formule VI:

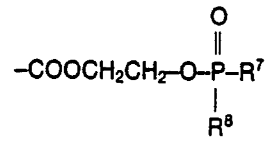
(VI)

dans lequel $R^7$ et $R^8$ représentent chacun, indépendamment l'un de l'autre, un alkyle contenant de 1 à 7 atomes de carbone, un radical de formule VII:

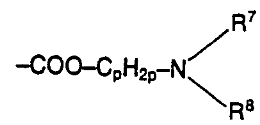
(VII)

dans lequel $R^7$ et $R^8$ ont les significations précédemment données et p désigne un nombre de 1 à 4, un radical de formule VIII:

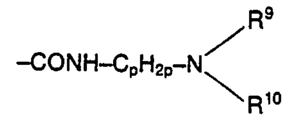
(VIII)

dans lequel $R^9$ et $R^{10}$ représentent chacun, indépendamment l'un de l'autre, un alkyle contenant de 1 à 4 atomes de carbone et p désigne un nombre de 1 à 4, ou un radical quaternisé correspondant à l'une des formules VII et VIII, le cation $M^{\oplus}$ dérivant d'une base hydrosoluble dont la force suffit pour neutraliser les radicaux sulfo et carboxy mentionnés et qui ne nuit pas à leur hydrophilie.

2. Copolymère réticulé selon la revendication 1, caractérisé en ce que, dans les groupements de liaison de formule I, $R^1$ et $R^2$ représentent chacun, indépendamment l'un de l'autre, l'hydrogène ou un alkyle contenant un ou deux atomes de carbone.

3. Copolymère réticulé selon l'une quelconque des revendications 1 et 2, caractérisé en ce que, dans les éléments constitutifs des chaînes de base qui répondent à la formule III, le radical alcoxycarbonyle que peuvent représenter $R^4$ et/ou $R^5$ contient de un à trois atomes de carbone dans sa partie alkyle, et les radicaux alkyles que peuvent représenter $R^6$, $R^7$ et/ou $R^8$ et $R^9$ et/ou $R^{10}$ contiennent un ou deux atomes de carbone.

4. Copolymère réticulé selon l'une quelconque des revendications 1 à 3, caractérisé en ce que les chaînes de base sont constituées d'éléments répondant à la formule IX:

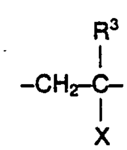
(IX)

dans laquelle $R^3$ représente l'hydrogène ou un radical méthyle et X représente: un radical carbamoyle $-CONH_2$, un radical de formule IV:

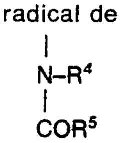
(IV)

dans lequel $R^4$ et $R^5$ représentent chacun, indépendamment l'un de l'autre, l'hydrogène, un méthyle ou un éthyle ou forment ensemble un radical triméthylène ou pentaméthylène, un radical carboxy ou l'un de ses sels de métaux alcalins ou d'ammoniums, un radical hydroxyalcoxycarbonyle dont la partie hydroxyalkyle contient 2 ou 3 atomes de carbone, de préférence 2, un radical sulfo, un radical sulfo-alkylamino-carbonyle dont la partie alkyle contient de 1 à 4 atomes de carbone, de préférence un radical de formule:

$$-CO-NH-C(CH_3)_2-CH_2-SO_3^{\ominus}M^{\oplus},$$

un radical phosphono, les radicaux sulfo et phosphono pouvant également être sous la forme de leurs sels de métaux alcalins ou d'ammoniums, ou un radical d'ester d'acide phosphonique de formule V:

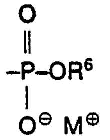

(V)

dans laquelle $R^6$ représente un alkyle contenant de 1 à 4 atomes de carbone, de préférence 1 ou 2.

5. Copolymère réticulé selon l'une quelconque des revendications 1 à 4, caractérisé en ce qu'il contient de 0,01 à 6% en poids, plus spécialement de 0,05 à 2% en poids, de groupements de liaison et de 99,99 à 94% en poids, plus particulièrement de 99,95 à 98% en poids, d'éléments constitutifs des chaînes de base.

6. Procédé pour préparer des copolymères réticulés selon l'une quelconque des revendications 1 à 5, procédé caractérisé en ce qu'on copolymérise des composés à insaturation éthylénique de formule IIIa:

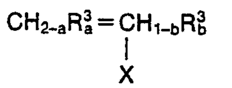

(IIIa)

devant constituer les chaînes de base, avec des monomères réticulants de formule Ia:

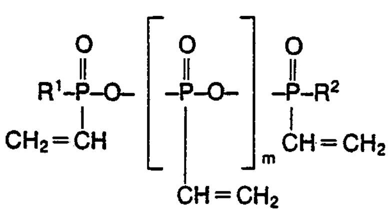

(Ia)

formules dans lesquelles $R^1$, $R^2$, $R^3$, X, a, b et m ont les significations précédemment données.

7. Application des copolymères réticulés selon l'une quelconque des revendications 1 à 5 pour la fabrication de revêtements et d'encapsulages solubles dans les acides.

8. Application des copolymères réticulés selon l'une quelconque des revendications 1 à 5 comme adjuvants de teinturerie.

9. Application des copolymères réticulés selon l'une quelconque des revendications 1 à 5 comme adjuvants pour diminuer la perte d'eau de boues de ciment.

10. Application des copolymères réticulés selon l'une quelconque des revendications 1 à 5 comme agents d'absorption pour des liquides aqueux.

**Revendications pour l'état contractant AT**

1. Procédé pour préparer des copolymères réticulés hydrophiles, procédé caractérisé en ce qu'on fait réagir de 0,01 à 30% en poids de comonomères réticulants répondant à la formule Ia:

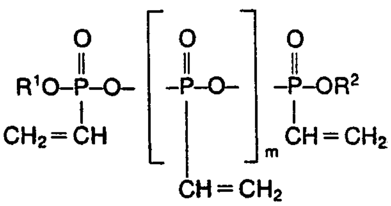

(Ia)

dans laquelle: $R^1$ et $R^2$ représentent chacun, indépendamment l'un de l'autre, l'hydrogène ou un alkyle contenant de 1 à 4 atomes de carbone et m désigne un nombre de 0 à 6, et de 99,99 à 70% en poids de composés éthyléniques, éléments constitutifs de chaînes de base, qui répondent à la formule IIIa:

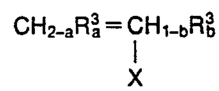

(IIIa)

dans laquelle: $R^3$ représente l'hydrogène ou un méthyle, a et b sont égaux chacun à 0 ou à 1, la somme (a+b) étant égale à 0 ou à 1, et X représente: un radical carbamoyle $-CONH_2$, un radical de formule IV:

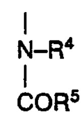

(IV)

dans lequel $R^4$ et $R^5$ représentent chacun, indépendamment l'un de l'autre, l'hydrogène, un méthyle ou un éthyle ou forment ensemble un radical triméthylène ou pentaméthylène, un carboxy ou un sel de celui-ci avec un cation $M^{\oplus}$, un alcoxycarbonyle dont la partie alkyle contient de 1 à 6 atomes de carbone, un hydroxyalcoxycarbonyle dont la partie hydroxyalkyle contient de 1 à 3 atomes de carbone, un radical N-(hydroxyméthyl)-carbamoyle $HOCH_2NH-CO-$ dont le radical hydroxyméthyle est éventuellement éthérifié par un alcanol contenant de 1 à 4 atomes de carbone, un alcanoylamino en $C_1$–$C_4$ qui porte éventuellement un radical hydroxyméthyle ou un alkyle contenant de 1 à 4 atomes de carbone, un radical cyano, un radical phényle ou benzyle éventuellement porteur d'un alkyle contenant de 1 à 4 atomes de carbone, d'un alcoxy contenant 1 ou 2 atomes de carbone, d'un halogène, d'un trifluorométhyle ou d'un nitro, un radical imidazolyle-1, un radical sulfo, un radical sulfoalkyl-aminocarbonyle dont la partie alkyle contient de 1 à 4 atomes de carbone, un radical phosphono, le radical sulfo et le radical phosphono pouvant également être sous la forme d'un sel avec un cation $M^{\oplus}$, un radical d'ester phosphonique de formule V:

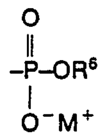

(V)

dans lequel $R^6$ représente un alkyle contenant de 1 à 4 atomes de carbone, un radical de formule VI:

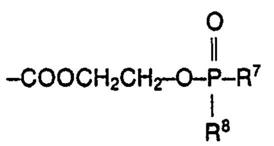

(VI)

dans lequel $R^7$ et $R^8$ représentent chacun, indépendamment l'un de l'autre, un alkyle contenant de 1 à 7 atomes de carbone, un radical de formule VII:

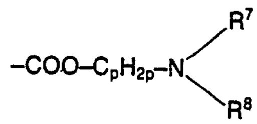

(VII)

dans lequel $R^7$ et $R^8$ ont les significations précédemment données et p désigne un nombre de 1 à 4, un radical de formule VIII:

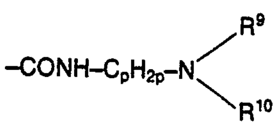

(VIII)

dans lequel $R^9$ et $R^{10}$ représentent chacun, indépendamment l'un de l'autre, un alkyle contenant de 1 à 4 atomes de carbone et p désigne un nombre de 1 à 4, ou un radical quaternisé correspondant à l'une des formules VII et VIII, le cation $M^{\oplus}$ dérivant d'une base hydrosoluble dont la force suffit pour neutraliser les radicaux sulfo et carboxy mentionnés et qui ne nuit pas à leur hydrophilie.

2. Procédé pour préparer des copolymères réticulés selon la revendication 1, procédé caractérisé en ce que, dans les comonomères de formule Ia, $R^1$ et $R^2$ représentent chacun, indépendamment l'un de l'autre, l'hydrogène ou un alkyle contenant un ou deux atomes de carbone.

3. Procédé pour préparer des copolymères réticulés selon l'une des revendications 1 et 2, procédé caractérisé en ce que, dans les comonomères de formule IIIa, le radical alcoxycarbonyle que peuvent représenter $R^4$ et/ou $R^5$ contient, dans sa partie alkyle, de un à trois atomes de carbone, et les radicaux alkyles que peuvent représenter $R^6$, $R^7$ et/ou $R^8$ et $R^9$ et/ou $R^{10}$ contiennent un ou deux atomes de carbone.

4. Procédé pour préparer des copolymères réticulés selon l'une quelconque des revendications 1 à 3, procédé caractérisé en ce qu'on choisit les comonomères de formule IIIa de telle façon que les chaînes de base des copolymères soient constituées d'éléments répondant à la formule IX:

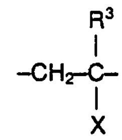

(IX)

dans laquelle $R^3$ représente l'hydrogène ou un radical méthyle et X représente: un radical carbamoyle $-CONH_2$, un radical de formule IV:

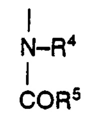

(IV)

dans lequel $R^4$ et $R^5$ représentent chacun, indépendamment l'un de l'autre, l'hydrogène, un méthyle ou un éthyle ou forment ensemble un radical triméthylène ou pentaméthylène, un radical carboxy ou l'un de ses sels de métaux alcalins ou d'ammoniums, un radical hydroxyalcoxycarbonyle dont la partie hydroxyalkyle contient 2 ou 3 atomes de carbone, de préférence 2, un radical sulfo, un radical sulfo-alkylamino-carbonyle dont la partie alkyle contient de 1 à 4 atomes de carbone, de préférence un radical de formule:

$$-CO-NH-C(CH_3)_2-CH_2-SO_3^{\ominus}M^{\oplus},$$

un radical phosphono, les radicaux sulfo et phosphono pouvant également être sous la forme de leurs sels de métaux alcalins ou d'ammoniums, ou un radical d'ester d'acide phosphonique de formule V:

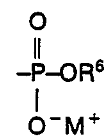

(V)

dans laquelle $R^6$ représente un alkyle contenant de 1 à 4 atomes de carbone, de préférence 1 ou 2.

5. Procédé pour préparer des copolymères réticulés selon l'une quelconque des revendications 1 à 4, procédé caractérisé en ce qu'on met en jeu de 0,01 à 6 parties en poids de comonomères de formule Ia, plus particulièrement de 0,05 à 2, et de 99,99 à 94 parties en poids de comonomères de formule IIIa, plus particulièrement de 99,95 à 98.

6. Application des copolymères réticulés préparés selon l'une quelconque des revendications 1 à 5 pour la fabrication de revêtements et d'encapsulages solubles dans les acides.

7. Application des copolymères réticulés préparés selon l'une quelconque des revendications 1 à 5 comme adjuvants de teinturerie.

8. Application des copolymères réticulés préparés selon l'une quelconque des revendications 1 à 5 comme adjuvants permettant de diminuer la perte d'eau de boues de ciment.

9. Application des copolymères réticulés préparés selon l'une quelconque des revendications 1 à 5 comme agents d'absorption pour des liquides aqueux.